# EUROPEAN PATENT APPLICATION

(11) **EP 4 397 681 A1**
(43) Date of publication of application: **10.07.2024**
(21) Application number: 22864469.6
(22) Date of filing: 29.08.2022
(51) Int. Cl.: C07K 16/00, C07K 17/00, G01N 33/531

(54) **ANTIBODIES, IMMOBILIZED ANTIBODIES USING SAME, ANTIBODY COMPOSITION, REAGENT FOR IMMUNOLOGICAL MEASUREMENT, IMMUNOLOGICAL MEASUREMENT METHOD, AND METHOD FOR IMPROVING ANTIGEN RESPONSIVENESS OF ANTIBODIES**

(30) Priority: 30.08.2021 JP 2021140250
(71) Applicant: EIKEN KAGAKU KABUSHIKI KAISHA, Tokyo 110-8408 (JP)
(72) Inventor: KOZUKA Naoyuki, Shimotsuga-gun, Tochigi 329-0114 (JP)
(74) Representative: J A Kemp LLP
(86) International application number: PCT/JP2022/032335
(87) International publication number: WO 2023/032886

(57) **Abstract**

An antibody for use in an immunoassay method, the antibody comprising an Fc region, wherein at least part of a glycan comprised in the Fc region has been removed.

## Description

### [Technical Field]

The present invention relates to an antibody, an immobilized antibody using the same, an antibody composition, a reagent for immunoassay, and an immunoassay method, as well as a method for improving reactivity of an antibody with an antigen.

### [Background Art]

It is known that proteins are subjected to various post-translational modifications after syntheses, and Glycan-modification is one of them. Glycan-modification of proteins starts from en bloc transfer from lipid intermediates in rough-surfaced endoplasmic reticulum and proceeds in the course of transition in a Golgi organ (NPL 1).

Antibodies are one of glycoproteins and are composed of Fab regions including variable regions comprising antigen-binding sites and Fc region which is a constant region. Typically, an N-linked glycan binds to the 297th asparagine residue of heavy chains present in the Fc regions of antibodies. It is known that N-linked glycans receive modifications from various enzymes, and are also affected by medium compositions and concentrations of dissolved oxygen, and thus become glycans having heterogeneous types and numbers of sugars to be added (NPLs 2, 3).

In addition, it is known that N-linked glycans affect thermal stabilities, antibody-dependent cellular cytotoxicity (ADCC) activities, and the like, and thus are one of important items that determine qualities in the field of antibody preparation (NPLs 4, 5). For this reason, techniques for making heterogeneous glycans homogeneous have been under development (NPL 6).

On the other hand, in the field of diagnostic agents as well, a method for enhancing the antigen binding activity of an antibody, that is, the reactivity of the antibody with an antigen by removing an N-linked glycan added to a complementarity determining region (also referred to as CDR) present in a variable region of the antibody have been found (PTL 1).

Normally, in the case of attempting to improve a diagnostic agent utilizing immunological reactions in order to detect a certain antigen, an antibody having a more excellent reactivity (binding force) to the antigen than antibodies that have already been used in diagnostic agents is demanded, and for example, methods that re-select an antibody having a stronger binding force to the antigen by immunizing an animal and methods that improve the binding force to the antigen by direct modification using genetic engineering procedures such as replacing an amino acid residue of a variable region of an antibody are employed.

However, whether or not an antibody having more excellent reactivity (binding force) to an antigen is obtained by these methods depend largely on chance.

In addition, since there have still been many unknowns on the effects of N-linked glycans, it is demanded to find out and control these.

### [Citation List]

### [Patent Literature]

[PTL 1] Japanese Patent No. 3439780

### [Non Patent Literatures]

[NPL 1] Kagaku To Seibutsu vol. 30, No. 4, p. 236-243, 1992
[NPL 2] Glycobiology 19, p. 936-949, 2009
[NPL 3] J. Biotechnol. 188, p. 88-96, 2014
[NPL 4] MAbs 11, p. 350-372, 2019
[NPL 5] J. Biol. Chem. 277, p. 26733-26740, 2002
[NPL 6] J. Biol. Chem. 291, p. 9356-9370, 2016

### [Summary of Invention]

### [Technical Problem]

The present invention has been made in view of the above-described problems of the conventional techniques, and an object thereof is to provide an antibody excellent in reactivity with an antigen in an immunoassay method, an immobilized antibody using the same, an antibody composition, a reagent for immunoassay, and an immunoassay method, as well as a method for improving reactivity of an antibody with an antigen.

### [Solution to Problem]

As a result of conducting earnest studies in order to achieve the above object, the present inventors have found that surprisingly it is possible to improve the reactivity of an antibody in an immunoassay method, particularly, the reactivity of an antibody when the antibody is supported on a carrier, by removing a glycan of an Fc region, which is a constant region, without conducting modification of variable regions as conventionally conducted, and eventually completed the present invention.

Specifically, the present invention encompasses the following aspects.
[1] An antibody for use in an immunoassay method, the antibody comprising an Fc region wherein at least part of a glycan comprised in the Fc region has been removed.
[2] The antibody according to [1], wherein the Fc region is a region derived from a mammal.
[3] The antibody according to [1] or [2], that is IgG, reduced IgG, or half IgG.
[4] The antibody according to any one of [1] to [3], wherein an amino acid residue at position 297 based on Kabat numbering in the Fc region is asparagine, and part of an N-linked glycan binding to position 297 based on Kabat numbering has been cleaved and removed.
[5] The antibody according to any one of [1] to [3], wherein an N-linked glycan has not bound to position 297 based on Kabat numbering in the Fc region.
[6] The antibody according to any one of [1] to [3], and [5], wherein an amino acid residue at position 297 based on Kabat numbering in the Fc region is asparagine, and an N-linked glycan has not bound to position 297 based on the Kabat numbering.
[7] The antibody according to [1] to [3], and [5], that is a recombinant antibody in which an amino acid residue at position 297 based on Kabat numbering in the Fc region is replaced with an amino acid other than asparagine, and an N-linked glycan has not bound to position 297 based on the Kabat numbering.
[8] The antibody according to [7], that is a recombinant antibody in which the amino acid residue at position 297 based on the Kabat numbering in the Fc region is replaced with one selected from the group consisting of serine, cysteine, alanine, leucine, isoleucine, valine, glycine, threonine, phenylalanine, tyrosine, methionine, tryptophan, glutamine, and methionine.
[9] The antibody according to [7] or [8], that is a recombinant antibody in which the amino acid residue at position 297 based on the Kabat numbering in the Fc region is replaced with cysteine, and an amino acid residue at position capable of forming a disulfide bond with the cysteine is also replaced with cysteine.
[10] The antibody according to [9], that is a recombinant antibody in which the amino acid residue at position 298 based on the Kabat numbering in the Fc region is replaced with cysteine.
[11] An immobilized antibody comprising: an insoluble carrier; and the antibody according to any one of [1] to [10] supported on the insoluble carrier.
[12] The immobilized antibody according to [11], wherein the insoluble carrier is at least one selected from the group consisting of a plate carrier, a membrane carrier, and a particle carrier.
[13] The immobilized antibody according to [11] or [12], wherein the insoluble carrier is at least one type selected from the group consisting of latex particles and gold colloid particles.
[14] An antibody composition comprising the antibody according to any one of [1] to [10] or the immobilized antibody according to any one of [11] to [13].
[15] A reagent for immunoassay, comprising the antibody according to any one of [1] to [10] or the immobilized antibody according to any one of [11] to [13] .
[16] An immunoassay method comprising a step of using the antibody according to any one of [1] to [10] or the immobilized antibody according to any one of [11] to [13] .
[17] A method for improving reactivity of an antibody with an antigen, comprising a step of removing, in an antibody comprising an Fc region for use in an immunoassay method, at least part of a glycan comprised in the Fc region.
[18] The method for improving reactivity of an antibody with an antigen according to [17], wherein the Fc region of the antibody is a region derived from a mammal.
[19] The method for improving reactivity of an antibody with an antigen according to [17] or [18], wherein the antibody is IgG, reduced IgG, or half IgG.
[20] The method for improving reactivity with an antigen according to any one of [17] to [19], comprising a step of removing all or part of an N-linked glycan at position 297 based on Kabat numbering in the Fc region.
[21] The method for improving reactivity of an antibody with an antigen according to [20], comprising a step of cleaving and removing all or part of an N-linked glycan at position 297 based on the Kabat numbering in the Fc region by means of enzyme treatment.
[22] The method for improving reactivity of an antibody with an antigen according to [20], comprising a step of replacing an amino acid residue at position 297 based on the Kabat numbering in the Fc region with an amino acid other than asparagine.
[23] The method for improving reactivity of an antibody with an antigen according to [22], comprising a step of replacing the amino acid residue at position 297 based on the Kabat numbering in the Fc region with one selected from the group consisting of serine, cysteine, alanine, leucine, isoleucine, valine, glycine, threonine, phenylalanine, tyrosine, methionine, tryptophan, glutamine, and methionine.
[24] The method for improving reactivity of an antibody with an antigen according to [22] or [23], further comprising: a step of replacing the amino acid residue at position 297 based on the Kabat numbering in the Fc region with cysteine and also replacing an amino acid residue at position capable of forming a disulfide bond with the cysteine with cysteine.
[25] The method for improving reactivity of an antibody with an antigen according to [24], comprising a step of replacing an amino acid residue at position 298 based on the Kabat numbering in the Fc region with cysteine.

### [Advantageous Effects of Invention]

The present invention makes it possible to provide an antibody excellent in reactivity to an antigen in immunoassay, an immobilized antibody using the same, an antibody composition, a reagent for immunoassay, and an immunoassay method, as well as a method for improving reactivity of an antibody with an antigen.

In addition, the present invention makes it possible, for example, without immunizing an animal to obtain an antibody having stronger binding force to an antigen in an immunoassay system or modifying a variable region of the antibody to improve the binding force to the antigen, to enhance the reactivity of antigen-antibody reaction, particularly reactivity when the antibody is supported on a carrier, by removing glycan comprised in an Fc region of the antibody. Moreover, the present invention also makes it possible to provide an antibody composition homogeneously comprising such an antibody excellent in reactivity. In addition, enhancement of a binding force of an antibody to an antigen by means of such removal of N-linked glycans is also one of means for significantly reducing time, work, cost required when an antibody having a more excellent reactivity is acquired.

### [Brief Description of Drawings]

[Fig. 1A] Fig. 1A is a schematic diagram of basic structures of N-linked glycans that can be present.
[Fig. 1B] Fig. 1B is a schematic diagram of other basic structures of glycans from which parts of N-linked glycans were cleaved and removed (partially removed glycans).
[Fig. 2A] Fig. 2A is a graph showing the result of TOF/MS analysis of an antibody (untreated antibody) before deglycosylation treatment, which was obtained in (2) of Test Example 1.
[Fig. 2B] Fig. 2B is a graph showing the result of TOF/MS analysis of an antibody (glycan removed antibody) after deglycosylation treatment, which was obtained in (2) of Test Example 1.
[Fig. 3] Fig. 3 is a graph showing a ΔOD value (ΔOD×10000) measured by using an untreated antibody-supporting latex emulsion or a glycan removed antibody-supporting latex emulsion at each antigen sample concentration (CRP (mg/dL)), which was obtained in (4) of Test Example 1.
[Fig. 4] Fig. 4 is a graph showing Ti values and thermal denaturation curves of an untreated antibody and a glycan removed antibody, which were obtained in (1) of Test Example 2.
[Fig. 5A] Fig. 5A is a graph showing the result of Biacore (registered trademark) measurement of the binding amount (RU) of an antigen when the untreated antibody or the glycan removed antibody was used, which was obtained in (2) of Test Example 2.
[Fig. 5B] Fig. 5B is a graph showing the result of Biacore measurement of the binding amount (RU) of an antigen when the untreated antibody or the glycan removed antibody was exposed to a latex support condition, which was obtained in (2) of Test Example 2.
[Fig. 6] Fig. 6 is a graph showing the result of Biacore measurement of the binding amount (RU) of an antigen when the untreated antibody-supporting latex emulsion or the glycan removed antibody-supporting latex emulsion was used, which was obtained in (3) of Test Example 2.
[Fig. 7A] Fig. 7A is a graph showing the result of TOF/MS analysis of an antibody A before deglycosylation treatment (untreated antibody A) (a) and the result of TOF/MS analysis of an antibody A after deglycosylation treatment (glycan removed antibody A) (b), which were obtained in (2) of Test Example 3.
[Fig. 7B] Fig. 7B is a graph showing the result of TOF/MS analysis of an antibody B (untreated antibody B) before deglycosylation treatment (a) and the result of TOF/MS analysis of an antibody B (glycan removed antibody B) after deglycosylation treatment (b), which were obtained in (2) of Test Example 3.
[Fig. 8] Fig. 8 is a graph showing ΔOD values (ΔOD×10000) measured by using mixture liquids I to III at each antigen sample concentration (ng/mL), which were obtained in (4) of Test Example 3.
[Fig. 9A] Fig. 9A is a graph showing Ti values and thermal denaturation curves of the untreated antibody A and the glycan removed antibody A, which were obtained in (1) of Test Example 4.
[Fig. 9B] Fig. 9B is a graph showing Ti values and thermal denaturation curves of the untreated antibody B and the glycan removed antibody B, which were obtained in (1) of Test Example 4.
[Fig. 10] Fig. 10 is a graph showing ΔOD values measured by using the untreated antibody A or the glycan removed antibody A as capturing bodies at each antigen solution concentration (ng/mL), which were obtained in (2) of Test Example 4.
[Fig. 11A] Fig. 11A is a graph showing the result of TOF/MS analysis of WT (a), N297S (b), and N297C-S298C (c), which was obtained in (2) of Test Example 5.
[Fig. 11B] Fig. 11B is an enlarged diagram of a range of 2080 to 3020 m/z in Fig. 11A.
[Fig. 11C] Fig. 11C is an enlarged diagram of a range of 1050 to 1250 m/z in Fig. 11A.
[Fig. 12] Fig. 12 is a graph showing Ti values and thermal denaturation curves of the Hybridoma-untreated antibody, the Hybridoma glycan removed antibody, WT, N297S, and N297C-S298C, which were obtained in (3) of Test Example 5.
[Fig. 13] Fig. 13 is a schematic diagram showing cleavage of a disulfide bond by using DTT.
[Fig. 14] Fig. 14 is a graph showing the result of TOF/MS analysis of reduced (+DTT) or non-reduced (-DTT) N297S (a, b) and reduced (+DTT) or non-reduced (-DTT) N297C-S298C (c, d), which was obtained in (4) of Test Example 5.
[Fig. 15] Fig. 15 is a graph showing ΔOD values (ΔOD×10000) measured by using the Hybridoma-untreated antibody, the Hybridoma glycan removed antibody, WT, N297S, or N297C-S298C at each antigen sample concentration (CRP (mg/dL)), which were obtained in (7) of Test Example 5.
[Fig. 16A] Fig. 16A is a graph showing the result of TOF/MS analysis (a range of 1500 to 3000 m/z) on the untreated antibody (a), the galactosidase-treated antibody (b), and the EndoF2-treated antibody (c), which was obtained in (3) of Test Example 6.
[Fig. 16B] Fig. 16B is a graph showing the result of TOF/MS analysis (a range of 1020 to 1600 m/z) on the untreated antibody (a) and the EndoF2-treated antibody (c), which was obtained in (3) of Test Example 6.
[Fig. 17] Fig. 17 is a graph showing ΔOD values (ΔOD×10000) measured obtained by using the untreated antibody, the galactosidase-treated antibody, the EndoF2-treated antibody, and the glycopeptidase-treated antibody at each antigen sample concentration (CRP (mg/dL)), which were obtained in (4) of Test Example 6.
[Fig. 18] Fig. 18 is a graph showing Ti values and thermal denaturation curves of the untreated antibody, the galactosidase-treated antibody, the EndoF2-treated antibody, the glycopeptidase-treated antibody, and N297S, which were obtained in (5) of Test Example 6.

### [Description of Embodiments]

Hereinafter, the present invention will be described in detail based on its preferred embodiments.

### <Antibody>

The present invention provides an antibody for use in an immunoassay method, the antibody comprising an Fc region wherein at least part of a glycan comprised in the Fc region has been removed.

In the present invention, the "immunoassay method" is a method for detecting, quantifying, and/or analyzing a test substance by utilizing an antigen-antibody reaction. The test substance is not particularly limited as long as the test substance can be detected by utilizing an antigen-antibody reaction, and includes low-molecular substances such as hormones and high-molecular substances such as proteins. A sample to be subjected to the immunoassay method is not particularly limited as long as the sample can comprise the test substance, and includes, for example, blood samples such as serum, plasma, and whole blood; and living organism-derived samples such as urine; stool; saliva; spinal fluid; oral mucosa; pharyngeal mucosa; and intestinal mucosa.

The immunoassay method includes, for example, an agglutination method (also referred to as agglutination turbidimetric immunoassay), an agglutination inhibition method, and nephelometric immunoassay using latex particles or particles of a metal colloid or the like; solid phase immunoassay such as ELISA; and immunochromatography, and the like. In addition, the solid phase immunoassay and the immunochromatography include, for example, an EIA method (enzyme immunoassay method) using an enzyme as a label (labeling substance); a RIA method (radioimmunoassay method) using a radioisotope as a label; a CLIA method (chemiluminescent immunoassay method) using a chemiluminescent compound as a label; combinations of these (for example CLEIA method (chemiluminescent enzyme immunoassay)); and the like, but are not limited to these. In addition, the immunoassay method according to the present invention may be a noncompetitive measurement method or a competitive measurement method.

The antibody of the present invention may be a monoclonal antibody or a polyclonal antibody, but is preferably a monoclonal antibody.

The class of the antibody of the present invention is not particularly limited, but is preferably one of IgG and IgM, and is more preferably IgG. The subclass is also not particularly limited, but is preferably one of IgG1, IgG2, IgG3, and IgG4, and is more preferably IgG1. The basic structure of the antibody of the present invention is a bivalent antibody composed of two light chains (L chains) and two heavy chains (H chains) (a Y-shaped four chain structure, preferably IgG and/or IgM), but the antibody of the present invention also encompasses a monovalent antibody (a reduced immunoglobulin or a half-immunoglobulin obtained by reducing an antibody of the basic structure and cleaving a disulfide bond linking two heavy chains (preferably, reduced IgG, reduced IgM, or half IgG)).

The origin of the antibody of the present invention is not particularly limited but is preferably an antibody in which at least an Fc region described later is derived from a mammal, and the mammal includes, for example, mouse, rat, rabbit, and human. The antibody comprising an Fc region derived from the mammal may be an antibody produced by the mammal, an antibody isolated from a hybridoma cell that produces the antibody, or a recombinant antibody prepared and produced based on an amino acid sequence of an antibody derived from the mammal in a genetic engineering manner. The antibody of the present invention may be, for example, a chimeric antibody of an Fc region derived from the mammal and Fab regions derived from another animal or a humanized antibody. In addition, the structure of the antibody of the present invention is not particularly limited as long as it comprises an Fc region described below. The structure of the antibody of the present invention preferably comprises Fab regions composed of a variable region, and the aspect of the Fab regions is not particularly limited, but can be prepared as appropriate in accordance with the object of the immunoassay.

The antibody of the present invention needs to comprise at least an Fc region. The "Fc region" is a region corresponding to a fragment on the C terminus side among two fragments obtained by separating the basic structure of an antibody composed of two types of polypeptide chains, two light chains and two heavy chains, that is, the above-described Y-shaped four chain structure by papain enzyme treatment, and a region obtained by the above-described enzyme treatment as an immunoglobulin molecule fragment (a portion corresponding to a vertical bar portion of the lower half of the above-described Y shape) in which a constant region of two heavy chains is linked by a disulfide bond. In the case where the antibody of the present invention is a bivalent antibody such as IgG or IgM, the Fc region according to the present invention comprises two Fc regions present respectively on two heavy chains in total. In the case where the antibody of the present invention is a monovalent antibody such as reduced IgG, reduced IgM, or half IgG, the Fc region according to the present invention indicates one Fc region present on one heavy chain.

The antibody of the present invention is an antibody in which at least part of a glycan comprised in the Fc region has been removed. In the present invention, the "antibody in which at least part of a glycan comprised in the Fc region has been removed" may be an antibody that has no glycan in the Fc region, or in the case where there are a plurality of glycans, an antibody that does not have at least one of them, or an antibody that does not have part of at least one glycan.

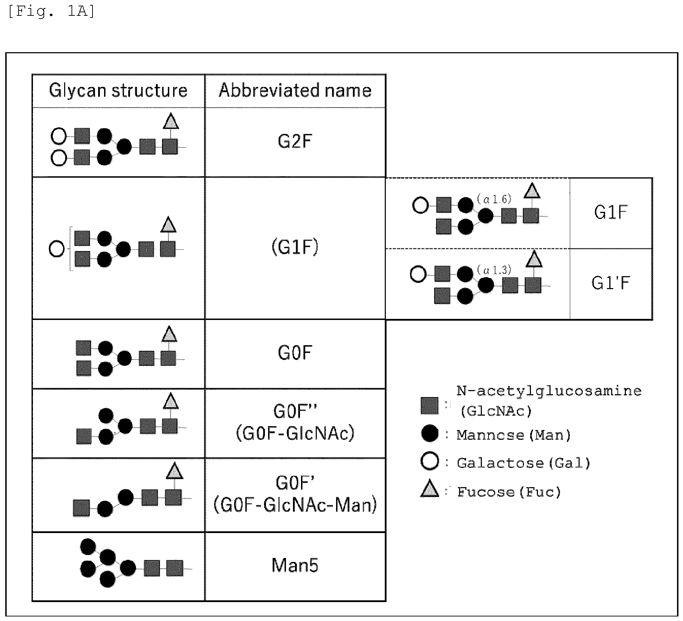

In the antibody of the present invention, a glycan that has been removed is preferably an N-linked glycan. The "N-linked glycan" according to the present invention indicates a glycan that binds to an amide nitrogen atom of a side chain of asparagine (Asn) of a consensus sequence (Asn-X-Ser/Thr: X is any amino acid other than Pro). Major basic structures of such N-linked glycans include, for example, glycan structures shown in Fig. 1A: G2F, G1F, G1'F (in the Specification, G1F and G1'F in the right column of Fig. 1A are collectively referred to as "G1F" unless otherwise noted), G0F, and Man5. For example, "GOF" has a biantennary chain in which N-acetylglucosamine to which fucose (Fuc) binds (-GlcNAc(Fc)-), N-acetylglucosamine (-GlcNAc-) and mannose (-Man<) bind to an amide nitrogen atom of a side chain of the asparagine in this order, and from the mannose, the mannose (-Man-) and N-acetylglucosamine (-GlcNAc) bind in this order. Besides, as N-linked glycans, glycan structures shown in Fig. 1A: G0F" and G0F' can also exist. Normally, in an Fc region of an antibody derived from a mammal, the asparagine is located at position 297 based on the Kabat numbering (that is, a sequence of Asn-X-Ser/Thr (glycan binding motif) is located at positions 297 to 299 based on the Kabat numbering), and an N-linked glycan binds to the asparagine residue.

The antibody of the present invention is preferably an antibody in which an N-linked glycan at position 297 based on the Kabat numbering has been removed (including partial removal of the N-linked glycan), and is preferably an antibody in which at least part of the N-linked glycan at position 297 based on the Kabat numbering has been removed (hereinafter sometimes referred to as a "glycan partially removed antibody") from the viewpoint of obtaining an excellent thermal stability. The glycan partially removed antibody is an antibody in which part of the N-linked glycan is cleaved and removed, and is an antibody in which asparagine is at position 297 based on the Kabat numbering, and more preferably an antibody comprising the above-described glycan binding motif.

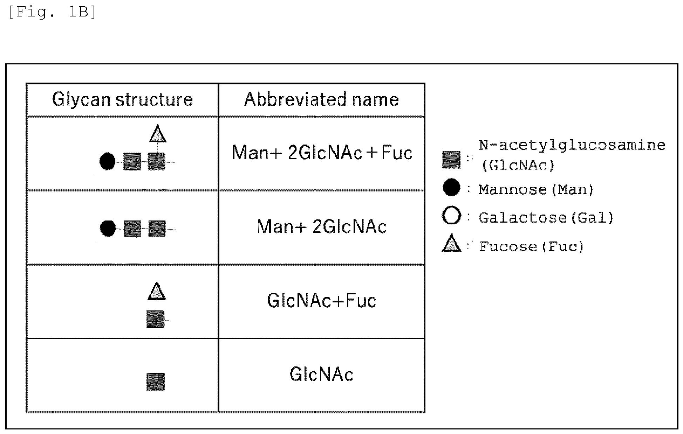

In a glycan partially removed antibody, the basic structure of a glycan in which part of a N-linked glycan has been cleaved and removed (hereinafter sometimes referred to as a "partially removed glycan") includes, for example, "GOF" as a partially removed glycan in which galactose (-Gal) at both terminuses of the biantennary chain of the "G2F" or one terminus of the biantennary chain of the "G1F" has been removed; "G0F"" as a partially removed glycan in which one N-acetylglucosamine (-GlcNAc) at one terminus of the biantennary chain of the "GOF" has been removed; and "G0F'" as a partially removed glycan in which N-acetylglucosamine (-GlcNAc) and mannose (-Man-) at one terminus of the biantennary chain of "GOF" have been removed. In addition, besides, glycan structures shown in Fig. 1B: Man+2GlcNAc+Fuc, Man+2GlcNAc, GlcNAc+Fuc, and GlcNAc are included. "Man+2GlcNAc+Fuc" is a partially removed glycan in which all sugars after mannose (-Man<) serving as a branching point of a biantennary chain have been removed from "G2F", "G1F", or "G0F"; "Man+2GlcNAc" is a partially removed glycan in which all sugars after mannose (-Man<) serving as a branching point of a biantennary chain have been removed from "Man5"; "GlcNAc+Fuc" is a partially removed glycan in which all sugars after N-acetylglucosamine (-GlcNAc(Fc)-) to which fucose (Fuc) binds have been removed from "G2F", "G1F", or "G0F"; and "GlcNAc" is a partially removed glycan in which all sugars after N-acetylglucosamine (-GlcNAc-) which binds to an amide nitrogen atom of a side chain of the above-described asparagine have been removed from "Man5". However, the partially removed glycan according to the present invention is not limited to these examples.

In addition, the antibody of the present invention is preferably an antibody in which at least all the N-linked glycan at position 297 based on the Kabat numbering has been removed, that is, an antibody in which no N-linked glycan binds to this position 297 based on the Kabat numbering (hereinafter sometimes referred to as a "glycan entirely removed antibody") from the viewpoint of reactivity to an antigen.

Note that in the Specification, regarding the number of an amino acid residue indicated "based on the Kabat numbering", the numbering of an amino acid residue of the variable region of the antibody is compliant with the numbering scheme made by Kabat et. al. (Kabat E. A. et al., Sequences of Proteins of Immunological Interest, 5th ed. Public Health Service, National Institutes of Health, Bethesda, MD (1991), NIH Publication No. 91-3242 (document I)), and the numbering of the constant region of the antibody, that is, the amino acid residue of the Fc region according to the present invention is compliant with the EU index made by Kabat et. al. The EU index is described in the above-described document by Kabat et. al.

For example, EU indexes of human IgG1, IgG2, IgG3, or IgG4 antibodies can be obtained from the IMGT website (http://www.imgt.org/IMGTScientificChart/Numbering/Hu_ IGHGnber.html). Amino acid sequences of heavy chains of these human IgG1, IgG2, IgG3, and IgG4 antibodies have high homology to each other, and, for example, as described in the above-described document I, at positions 297 to 299 based on the Kabat numbering, sequences of Asn-Ser-Thr (glycan binding motif) are located in common.

The antibody of the present invention in which at least part of a glycan comprised in an Fc region has been removed can be obtained, for example, by a method including performing an enzyme treatment on an antibody comprising an Fc region to cleave and remove all or part of the glycan of the Fc region from the antibody, as an enzyme-treated antibody in which at least part of the glycan (preferably all or part of an N-linked glycan has been cleaved and removed by the enzyme treatment. In addition, for example, the antibody of the present invention can also be obtained by a genetic engineering procedure modifying the consensus sequence specific to addition of an N-linked glycan comprised in the Fc region of an antibody as a recombinant antibody which is not subjected to N-linked glycan-modification in an antibody-producing cell (also referred to as a host cell). It can be confirmed that the glycan has been removed from the antibody by these methods from events that the peak of the glycan (preferably, the peak of the N-linked glycan at position 297 based on the Kabat numbering) which was observed before a deglycosylation treatment disappears and/or the peak of a peptide fragment in which the glycan has been removed or a partially removed glycan which was not observed before the deglycosylation treatment appears, by analysis of peptide fragments and glycans of the antibody using TOF/MS analysis, for example.

In the case where all of an N-linked glycan is cleaved and removed as the method for cleaving and removing a glycan by the above-described enzyme treatment, for example, a method using glycopeptidase F (also called Peptide-N⁴-(N-acetyl-β-glucosaminyl)asparagine amidase, Peptide N-Glycosidase F, or PNGaceF) may be employed. This enzyme is an oligosaccharide-cleaving enzyme (glycosidase) specifically cleaving an N-linked glycan, and is often used in the analysis of N-linked glycan-modification of proteins, and commercially-available ones can be obtained as appropriate. Enzyme-treated antibodies that can be obtained by the glycopeptidase F treatment (in the Specification, sometimes referred to as a "glycopeptidase-treated antibodies") include, for example, antibodies (glycan entirely removed antibodies) in which all of an N-linked glycan has been cleaved and removed.

In addition, in the case of cleaving and removing part of an N-linked glycan as another method for cleaving and removing a glycan by using the above-described enzyme treatment, for example, a method using galactosidase can also be employed. This enzyme is classified into α-galactosidase, which hydrolyzes α-galactoside, and β-galactosidase, which hydrolyzes β-galactoside, and is an enzyme that specifically cleaves glycosidic bonds between galactose residues and other sugar residues. The origin or the like of such galactosidase is not particularly limited, and a commercially-available one (for example, β1-4 Galactosidase (produced by QA-bio), β-galactosidase GalactEXO (produced by Genovis)) can be employed as appropriate. Enzyme-treated antibodies obtained by the above-described galactosidase treatment (in the Specification, sometimes referred to as "galactosidase-treated antibodies") include, for example, antibodies having glycans of "GOF" at position 297 based on the Kabat numbering as antibodies (glycan partially removed antibodies) in which part of the N-linked glycans has been cleaved and removed, which are obtained by β-galactosidase treatment.

In addition, in the case of removing part of an N-linked glycan as another method for cleaving and removing a glycan by using the above-described enzyme treatment, for example, a method using EndoF2 (produced by New England Biolabs) can also be employed. EndoF2 is a recombinant endoglycosidase that cleaves N-linked biantennary glycans or high-mannose glycans. Enzyme-treated antibodies that can be obtained by the EndoF2 treatment (in the Specification, sometimes referred to as "EndoF2-treated antibodies") include, for example, antibodies having glycans of "GlcNAc+Fuc" or "GlcNAc" at position 297 based on the Kabat numbering as antibodies in which part of N-linked glycans have been cleaved and removed (glycan partially removed antibodies).

Moreover, in the case of removing part of an N-linked glycan as another method for cleaving and removing a glycan by using the above-described enzyme treatment, for example, a method using mannosidase can be employed. Mannosidase (also called α-mannosidase, α-D-mannoside mannohydrolase) is exoglycosidase that cleaves the α form of mannose. The origin or the like of such mannosidase is not particularly limited, and a commercially-available one (for example, α(1-2,3,6)-mannosidase (produced by Ludger Ltd.), α(1,6)core mannosidase (produced by Ludger Ltd.), or α1-6 Mannosidase (produced by New England Biolabs)) can be employed as appropriate. Enzyme-treated antibodies that can be obtained by the above-described mannosidase treatment (in the Specification, sometimes referred to as "mannosidase-treated antibodies") include, for example, antibodies having glycans of "Man+2GlcNAc+Fuc" or "Man+2GlcNAc" at position 297 based on the Kabat numbering as antibodies (glycan partially removed antibodies) in which part of the N-linked glycans has been cleaved and removed.

As the method for cleaving and removing a glycan by using the above-described enzyme treatments, conventionally known methods or methods based on the conventionally known methods can be employed as appropriate depending on the type of each enzyme. In addition, two or more of the above-described enzyme treatments may be conducted simultaneously or sequentially as appropriate in combination.

In the case of removing all of an N-linked glycan as a method for removing a glycan by using the above-described genetic engineering procedure, for example, a method including, based on the amino acid sequence of the Fc region of the antibody from which a glycan is to be removed, introducing a vector expressing an antibody in which a consensus sequence (preferably, asparagine (Asn) at position 297 based on the Kabat numbering) comprised in the Fc region is deleted or replaced with another amino acid into a host cell (antibody-producing cell) to express a recombinant antibody may be employed. As the method for modifying (deleting or replacing) an amino acid by the above-described genetic engineering procedure, conventionally known methods or methods based on the conventionally known methods can be employed as appropriate. For example, publicly-known methods such as site-directed mutagenesis method (Kunkel et. al., (Proc. Natl. Acad. Sci. USA (1985) 82, p. 488-492)), overlap extension PCR can be employed as appropriate. The amino acid sequence of the Fc region of the target antibody can be obtained, for example, from a publicly-known database, for example, GenBank (http://www.ncbi.nlm.nih.gov) or the like. An amino acid sequence of the Fab regions to be combined with the Fc region can be employed as appropriate depending on the object of the immunoassay as mentioned above. In addition, the primer or the like used for modifying the amino acid can be designed as appropriate based on the amino acid sequence of the target antibody and the target modification by using conventionally known methods or methods based on the conventionally known methods.

In the case where the antibody of the present invention is a recombinant antibody in which all of a glycan has been removed by the genetic engineering procedure, it is more preferable that the recombinant antibody be a recombinant antibody in which an amino acid residue at position 297 based on the Kabat numbering in the Fc region has been replaced with an amino acid other than asparagine (glycan entirely removed antibody). The amino acid to be replaced other than asparagine is not particularly limited, but is preferably a neutral amino acid, and may be, for example, one selected from the group consisting of serine, cysteine, alanine, leucine, isoleucine, valine, glycine, threonine, phenylalanine, tyrosine, methionine, tryptophan, glutamine, and methionine, is more preferably one selected from the group consisting of serine, cysteine, and glutamine, and is further preferably serine or cysteine.

The antibody of the present invention may be one in which an amino acid other than the amino acid at position 297 based on the Kabat numbering has been further modified. Such modification includes replacement, deletion, insertion, and/or addition of one or a plurality of amino acids as long as such modification does not hinder the advantageous effects of the present invention, and the "plurality" preferably indicates an integer of 20, 19, 18, 17, 16, 15, 14, 13, 12, 11, 10, 9, 8, 7, 6, 5, 4, 3, or 2. Such modification includes, for example, replacement of arbitrary one or two amino acids with cysteine for introducing a disulfide bond; deletion of at least one (preferably both)of amino acids at position 298 and position 299 based on the Kabat numbering; replacement of an amino acid at position 298 based on the Kabat numbering with proline; and replacement of the amino acid at position 299 based on the Kabat numbering with an amino acid other than serine and threonine.

Among the above-described modifications, in the present invention, replacement of one or two amino acids with cysteine for introducing a disulfide bond is preferable. Replacement of two amino acid residues (for example, positions 297 and 298, positions 298 and 299, or positions 299 and 300) out of positions 297, 298, 299, and 300 based on the Kabat numbering in the Fc region with cysteine is more preferable, and removal (removal of all) of the above-described N-linked glycan by replacement of the amino acid residue at position 297 with cysteine, and replacement, with cysteine, of one amino acid residue at a position which can form a disulfide bond with the cysteine thus replaced at position 297 are particularly preferable. The present inventors newly found that there is a case where the thermal stability of the CH2 region in the Fc region decreases due to removal of the above-described N-linked glycan depending on an antibody, and in particular, the thermal stability particularly tends to decrease due to removal of the N-linked glycan by a genetic engineering procedure (for example, recombination of replacement of an amino acid residue at position 297 based on the Kabat numbering with an amino acid other than asparagine), but found that the decrease of the thermal stability of the CH2 region due to removal of the glycan can be suppressed by introducing such a disulfide bond.

The amino acid residue at a position that can form a disulfide bond with cysteine replaced at position 297 based on the Kabat numbering includes amino acid residue and the like at positions 296, 298, and 299 based on the Kabat numbering in the Fc region, and is more preferably the amino acid residue at position 298 based on the Kabat numbering.

The antibody of the present invention is excellent in reactivity to an antigen, and particularly reactivity when the antibody is supported on a carrier (for example, an insoluble carrier described below), because all or part of a glycan comprised in the Fc region has been removed. In the present invention, the "reactivity" of the antibody to an antigen has the same meaning as the binding force to the antigen or antigen binding activity, and is used as a term representing either or both of the affinity representing the binding strength between the antigen and the antibody and the binding activity.

In the present invention, for the reactivity of the antibody to an antigen, the degree of binding reaction between the antigen and the antibody, which is formed by specific interaction between the antigen and the antibody, that is, the magnitude of the reaction (or also referred to as a binding amount) can be used as an index. Specifically, indexes that can be used include, for example, an amount of change in absorbance which is generated by binding reaction between the antigen and the antibody in the case where the immunoassay method is an agglutination method or an agglutination inhibition method, an amount of change in scattered light in the case of a nephelometric immunoassay, a measured value of a characteristic absorption wavelength generated by a change in color production of a substrate due to the activity of an enzyme as a labeling substance in the case of the EIA method, a measured value of radiation dose of a radiation species specific to a radioisotope as a labeling substance in the case of the RIA method, and an amount of change in absorbance, reflected light intensity, or fluorescence wavelength derived from a labeling substance in the case of the immunochromatography, and the like. The above-described amount of change is indicated by a measured value of absorbance generated due to binding reaction between the antigen and the antibody, a measured value of a characteristic absorbance, reflected light intensity, or fluorescence wavelength derived from a labeling substance, or the like, and may be, for example, any of an amount of change in single wavelength (Δ value), a difference between two wavelengths (between a main wavelength and a sub wavelength) or a ratio therebetween (an absorbance ratio of a main wavelength as a numerator and a sub wavelength as a denominator), or an amount of change of any of these.

From the viewpoint that the reactivity with an antigen is more excellent, as the antibody of the present invention, a glycan entirely removed antibody is preferable to a glycan partially removed antibody. In the case of a glycan entirely removed antibody, an enzyme-treated antibody in which all of an N-linked glycan has been cleaved and removed (for example, the above-described glycopeptidase-treated antibody) is preferable. In the case of a glycan partially removed antibody, one in which a glycan binding to position 297 based on the Kabat numbering is shorter is preferable, and, for example, an antibody that has "GlcNAc+Fuc" or "GlcNAc" at position 297 based on the Kabat numbering (for example, the above-described EndoF2-treated antibody) is preferable.

On the other hand, from the viewpoint that the thermal stability is more excellent, as the antibody of the present invention, a glycan partially removed antibody is preferable to a glycan entirely removed antibody. In the case of a glycan entirely removed antibody, an enzyme-treated antibody in which all of an N-linked glycan has been cleaved and removed (for example, the above-described glycopeptidase-treated antibody) is preferable. In the case of a glycan partially removed antibody, one in which a glycan binding to position 297 based on the Kabat numbering is longer is preferable, and, for example, an antibody having "GOF" at position 297 based on the Kabat numbering (for example, the above-described galactosidase-treated antibody) is preferable.

In addition, from the viewpoint of thermal stability and supply stability, as the antibody of the present invention, an enzyme-treated antibody in which all or part of an N-linked glycan has been cleaved and removed, that is, an antibody in which asparagine is located at position 297 based on the Kabat numbering (more preferably, an antibody comprising the above-described glycan binding motif) is preferable to a recombinant antibody. On the other hand, from the viewpoint of reactivity, production cost, and suppression of variations among antibodies, a recombinant antibody, that is, a recombinant antibody in which position 297 based on the Kabat numbering is replaced with an amino acid other than asparagine is preferable to an enzyme-treated antibody.

### <Method for Improving Reactivity of Antibody with Antigen>

As described above, according to the present invention, it is possible to improve reactivity of antigen-antibody reaction, particularly reactivity when the antibody is supported on a carrier, by removing part or all of a glycan comprised in an Fc region of the antibody. Hence, the present invention also provides a method for improving reactivity of an antibody with an antigen, comprising a step of removing, in an antibody comprising an Fc region for use in an immunoassay method, at least part of a glycan comprised in the Fc region. This method for improving reactivity of an antibody with an antigen makes it possible to obtain the antibody of the present invention. In addition, according to the present invention, it is also possible to obtain the antibody (antigen reactivity-improved antibody) of the present invention or an antibody composition uniformly comprising the antibody of the present invention. That is, the present invention also provides a method for producing an antibody or an antibody composition, comprising a step of removing, in an antibody comprising an Fc region for use in an immunoassay method, at least part of a glycan comprised in the Fc region.

An antibody (an antibody before removal of a glycan, hereinafter also referred to as an "untreated antibody") used in the method for improving reactivity of an antibody with an antigen and the method for producing an antibody or an antibody composition of the present invention (hereinafter sometimes referred to collectively as the " method of the present invention") is not particularly limited as long as the antibody comprises a glycan in an Fc region. The preferable form is as described in terms of the above-described antibody of the present invention except that all of the glycan to be removed by the enzyme treatment or the genetic engineering procedure are not removed. The untreated antibody is particularly preferably an antibody comprising an N-linked glycan in the Fc region, preferably, at position 297 based on the Kabat numbering. Such an antibody is preferably an antibody in which the Fc region is derived from a mammal, is more preferably IgG, and is typically IgG1. The untreated antibody can be produced as appropriate by a publicly-known method or a method based on the publicly-known method, and a commercially-available one can also be used as appropriate.

In the method of the present invention, the step of removing at least part of a glycan comprised in the Fc region of the untreated antibody is not particularly limited, but includes, for example, a step of removing all or part of a glycan by the enzyme treatment, and a step of removing all of a glycan by the genetic engineering procedure. Specific approaches in these steps are as described in terms of the above-described antibody of the present invention, including preferable forms.

The method of the present invention may comprise a step of supporting the obtained antibody of the present invention on an insoluble carrier described below, as necessary. In this way, an immobilized antibody and an antibody composition comprising the immobilized antibody, which are described below, can be obtained. The method for supporting the antibody on an insoluble carrier is as described in terms of an immobilized antibody described below.

### <Immobilized Antibody>

The antibody of the present invention may be obtained as an immobilized antibody in which the antibody is supported on an insoluble carrier as one embodiment. Hence, the present invention also provides an immobilized antibody comprising: an insoluble carrier; and the antibody of the present invention (also including an antibody obtained by the above-described method for improving reactivity of an antibody with an antigen of the present invention) supported on the insoluble carrier. The present inventors found that the antibody of the present invention tends to be more excellent in reactivity when the antibody is supported on an insoluble carrier. It is surmised that since part or all of a glycan has been removed, the orientation of the CH2 region of the Fc region is enhanced, so that the binding capability between the insoluble carrier and the antibody, which is caused mainly by hydrophobic interaction, is improved.

In the present invention, the "insoluble carrier" is not particularly limited as long as the antibody of the present invention can be immobilized and supported on the insoluble carrier and the insoluble carrier is insoluble in water under ordinary temperature and ordinary pressure, and the "insoluble carrier" includes insoluble carriers conventionally known in immunoassay methods given in terms of the above-described antibody of the present invention, and the like.

More specifically, the insoluble carrier includes, for example, particle carriers including latex particles such as polystyrene latex particles and polyethylene latex particles, metal colloid particles such as gold colloid particles, gelatin particles, and magnetic particles; plate carriers such as a well plate made of polystyrene; and membrane carriers consisting of fibers of nitrocellulose, polyethylene, polyethylene terephthalate, nylons, glass, cellulose, and the like, and any of artificial materials, natural materials, and mixed materials of these may be used as long as the material is used in general. Among these, the insoluble carrier according to the present invention is preferably at least one type selected from the group consisting of latex particles and gold colloid particles from the viewpoint that these are more excellent in reactivity of the supported antibody with an antigen.

In the present invention, the "support" has the same meaning as immobilization (also referred to as fixing), and as the method for the support, a method used in general may be conducted, and includes, for example, a physical adsorption method and a chemical bonding method. The removal of a glycan comprised in the Fc region of the antibody to be supported on the insoluble carrier may be conducted before or after the supporting of the antibody on the insoluble carrier. In addition, the antibody of the present invention to be supported on the insoluble carrier is preferably one type, but may be a combination of two or more.

### <Antibody Composition>

The present invention also provides an antibody composition comprising: the above-described antibody of the present invention (including an antibody obtained by the method for improving reactivity of an antibody with an antigen of the present invention) or the immobilized antibody of the present invention. The antibody composition of the present invention may comprise one type of the antibody of the present invention or the immobilized antibody of the present invention, or may comprise two or more types thereof in combination, and may comprise the antibody of the present invention and the immobilized antibody of the present invention in combination.

In general, even antibodies consisting of the same amino acid sequence have variations in types of glycans comprised in the Fc region among individual antibodies. For this reason, antibody compositions comprising these antibodies have heterogeneous types of glycans. On the other hand, in the present invention, even in the case where the antibody of the present invention is an antibody having a glycan (preferably, the above-described glycan partially removed antibody), it is possible to provide an antibody composition comprising an antibody having the same type of glycan as the antibody with a high purity, that is, with a high homogeneity, by deglycosylation treatment (for example, the above-described enzyme treatment). Hence, the antibody composition of the present invention is excellent in reactivity with an antigen and thermal stability.

The antibody composition of the present invention includes an aspect comprising an antibody in which a glycan has not been removed (untreated antibody) as an antibody besides the antibody of the present invention. As such an antibody composition having a high homogeneity, in the case where the antibody of the present invention is an antibody in which part of an N-linked glycan binding to position 297 based on the Kabat numbering has been cleaved and removed, and is, for example, a glycan partially removed antibody having "GOF" as a glycan in which galactose (-Gal) at one or both terminuses of a biantennary chain has been removed from "G2F" and/or "G1F", the content of galactose in a glycan comprised in the Fc region of the antibody in the composition may be 53% by mass or less, preferably 50% by mass or less, more preferably 30% by mass or less, and further preferably 15% by mass or less in a ratio of the antibody amount having a glycan comprising galactose to the total antibody amount (antibody amount comprising a glycan amount, or in the case where there are both an antibody that has a glycan and an antibody that does not have a glycan as antibodies, a total amount of these, the same applies below).

In addition, in the case where the antibody composition of the present invention is an aspect comprising an antibody in which a glycan has not been removed (untreated antibody) besides the antibody of the present invention, the diversity index (the Simpson's diversity index obtained from S: the number of types of glycans binding to position 297 based on the Kabat numbering (in the case where an antibody in which a glycan do not bind to the position 297 is comprised, this antibody is also counted as one type) and Pi: the relative priority of the glycan removed antibody (the amount of the glycan removed antibody/the total antibody amount)) of the glycan removed antibody (the glycan partially removed antibody and/or the glycan entirely removed antibody of the present invention) may be set to 0.73 or less, preferably 0.3 or less, and more preferably 0.1 or less. In addition, the relative priority (Pi) of the glycan removed antibody may be set to 0.15 or more, preferably 0.5 or more, and more preferably 0.8 or more.

Note that the content of galactose in the total mass of the glycan comprised in the Fc region of an antibody comprised in the antibody composition, the number of types of glycans binding to position 297 based on the Kabat numbering, and the relative priority of each antibody can be obtained, for example, by analysis of peptide fragments and the glycan of the antibody using the above-described TOF/MS analysis, and in this case, as each antibody amount, a signal-to-noise ratio (S/N) can be used.

The antibody composition of the present invention may further comprise, for example, a solvent such as water or a publicly-known buffer solution (sodium phosphate buffer, MES, Tris, CFB, MOPS, PIPES, HEPES, tricine buffer, bicine buffer, glycine buffer, or the like); and a surfactant.

### <Immunoassay method>

The present invention also provides an immunoassay method comprising a step of using the antibody of the present invention (including an antibody obtained by the method for improving reactivity of an antibody with an antigen of the present invention) or the immobilized antibody of the present invention (the antibody and the immobilized antibody also include those comprised in the antibody composition of the present invention).

The immunoassay method is as described in terms of the immunoassay method of an antibody of the present invention. In the case of using an immobilized antibody supported on a particle carrier such as latex particles or metal colloid particles as the antibody of the present invention, for example, it is possible to employ an agglutination method, an agglutination inhibition method, or a nephelometric immunoassay. In the case of using an immobilized antibody supported on a particle carrier such as magnetic particles or on a plate carrier, for example, it is possible to employ a solid phase immunoassay such as ELISA. In the case of using an immobilized antibody supported on a membrane carrier, for example, it is possible to employ an immunochromatography.

For example, as an aspect of the agglutination method includes a method comprising a step of bringing a sample and an immobilized antibody comprising a particle carrier and the antibody of the present invention supported on the particle carrier. The sample is as described in terms of the above-described antibody of the present invention. In the case where an antigen is present as a test substance in the above-described sample, since the particle carrier is agglomerated, it is possible to measure the presence or absence of the test substance and the amount of the test substance by detecting a signal derived from the agglomeration.

In addition, for example, an aspect of the above-described ELISA method includes sandwich ELISA comprising a step of bringing the sample, a capturing body, and a labeled body into contact with one another. The sandwich ELISA includes a forward sandwich method which is a two-step method (a method successively conducting reaction of a capturing body and a test substance in a sample, and reaction of the test substance bound to a capturing body and a labeled body), a reverse sandwich method (a method reacting in advance a labeled body and a test substance in a sample, and reacting a complex thus generated with a capturing body), and a one step method (a method conducting reactions of a test substance in a sample, a capturing body, and a labeled body in one step simultaneously). Any of these can be employed. In the case where an antigen is present as a test substance in the sample, since a complex of the capturing body-the test substance (antigen)-the labeled body is formed, it is possible to measure the presence or absence and the amount of the test substance by detecting a signal derived from the labeled body.

In the above-described sandwich ELISA, the capturing body includes the insoluble carrier, and a first molecule which is supported on the insoluble carrier and capable of binding to a test substance, and the labeled body includes a labeling substance and a second molecule which is capable of binding to the test substance. The labeling substance includes an enzyme, a luminescent substance, a fluorescent substance, a radioactive substance, gold colloid particles and the like.

Although the antibody of the present invention may be included in any of the capturing body and the labeled body as the first molecule and/or the second molecule which are capable of binding to the test substance, it is particularly preferable that the antibody of the present invention be included in both of them. In the case where antibody of the present invention is included in both of them, the first molecule and the second molecule may be the same (for example, both are an antibody A from which a glycan has been removed) or different (for example, one is an antibody A from which a glycan has been removed, and the other is an antibody B from which a glycan has been removed), and are preferably such that the recognized sites of an antigen do not overlap each other. In the case where the antibody of the present invention is included in one of the capturing body or the labeled body, a molecule that is capable of binding to the test substance includes a molecule that is capable of binding to a complex of the antibody of the present invention and a test substance, and includes, for example, antibodies that are capable of binding to a test substance other than the antibody of the present invention; binding proteins such as protein A, protein G, and protein L; avidins; lectins; glycan receptors, and the like.

In the immunoassay method of the present invention, the above-described detected signal can be made into the amount of the test substance by semi-quantifying or quantifying the amount of the the signal as necessary. The "signal" includes color reaction (color production), quenching, reflected light, luminescence, fluorescence, radioactive ray by radioisotope, and the like, and besides those which can be recognized by naked eyes, include those which can be recognized by a detection method or apparatus depending on the type of the signal. In the present invention, as the amount of the test substance, the amount may be calibrated from a calibration curve or the like using a reference sample, or the amount of the signal may be used as the amount of the test substance (for example, amount of an antigen) as it is.

In the immunoassay method of the present invention, the sample may be used as being diluted with a diluted solution. In addition, to the reaction system of the sample with the immobilized antibody, the capturing body and/or the labeled body, a reaction buffer may be added as appropriate. These diluted solution and reaction buffer are not particularly limited, but include, for example, each independently, publicly-known buffer solutions (sodium phosphate buffer, MES, Tris, CFB, MOPS, PIPES, HEPES, tricine buffer, bicine buffer, glycine buffer and the like), and each independently, a reaction modifier (a surfactant or the like) or the like may also be added thereto.

### <Reagent for Immunoassay>

The present invention also provides a reagent for immunoassay for use in the above-described immunoassay method, comprising the antibody of the present invention (also including antibodies obtained by the method for improving reactivity of an antibody with an antigen of the present invention) or the immobilized antibody of the present invention.

The reagent for immunoassay of the present invention may be one consisting of only at least one selected from the group consisting of the antibody and the immobilized antibody of the present invention, or may be the antibody composition of the present invention, but may further comprise another component as appropriate as long as the component does not hinder the advantageous effects of the present invention. Such other component includes solvents such as water, buffer solutions (for example, the above-described publicly-known buffer solutions); surfactants, and the like.

In the case where the reagent for immunoassay of the present invention is a composition comprising the above-described other component, the content of the antibody of the present invention in the composition is not particularly limited and can be adjusted as appropriate.

The reagent for immunoassay of the present invention may be made into a kit by being combined with a configuration that should be included in an immunoassay method such as the agglutination method, the agglutination inhibition method, the nephelometric immunoassay, the ELISA method, or the immunochromatography, for example. The configuration comprised in such a kit includes, besides the reagent for immunoassay of the present invention, for example, reference samples (standard sample reagents, each concentration), control reagents, the above-described diluted solutions, the above-described reaction buffers, cleaning liquids, and the like.

### [Examples]

Hereinafter, the present invention will be more specifically described using Examples, but the present invention is not limited to these Examples.

### (Test Example 1) Evaluation of reactivity of mouse anti-human CRP monoclonal antibodies (enzyme-treated antibodies) from which glycans were removed by using an enzyme (glycopeptidase F treatment)

### (1) Preparation of antibodies from which glycans were removed

To 1 mg of a mouse anti-human CRP (C-reactive protein) monoclonal antibody (IgG1), 1 µL of glycopeptidase F with 500 µU/µL (produced by Takara Bio Inc.) was added, followed by keeping at 37°C for 18 hours to prepare a mouse anti-human CRP monoclonal antibody subjected to a deglycosylation treatment (hereinafter referred to as a "glycan removed antibody").

### (2) Glycan Analysis of antibodies

First, to 1 µg of the glycan removed antibody prepared in (1), 10 µL of 8 M Urea-20 mM ammonium bicarbonate (pH 8.0) and 1 µL of 100 mM DTT were added, followed by keeping at 70°C for 3 minutes. Subsequently, 90 µL of 20 mM ammonium bicarbonate (pH 8.0) and 1 µL of 500 µg/mL Tripsin solution were added, followed by keeping at 37°C for 5 minutes, and further 1 µL of 10% trifluoroacetic acid was added to obtain a prepared solution. The prepared solution thus obtained was put into C18 ziptip (produced by Merck KGaA), and was eluted with 9% acetonitrile/0.1% trifluoroacetic acid. To the eluate thus obtained, an equal amount of HCCA (produced by Bruker) saturated using 100% acetonitrile was added, followed by mixing to prepare a sample solution. The sample solution thus obtained was subjected to MALDI-TOF/MS (produced by Bruker). In addition, as a comparison subject, a mouse anti-human CRP monoclonal antibody (hereinafter referred to as an "untreated antibody") which was not subjected to the deglycosylation treatment of (1) was treated in the same manner and was subjected to MALDI-TOF/MS.

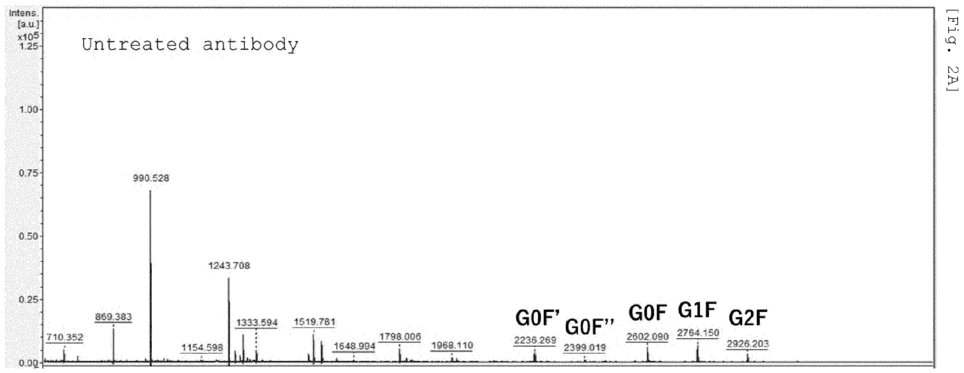

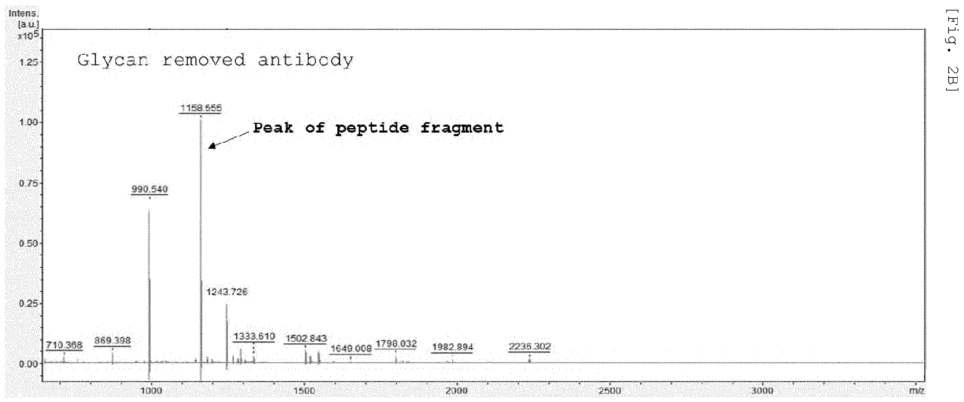

The result of TOF/MS analysis of the antibody before the deglycosylation treatment (untreated antibody) is shown in Fig. 2A, and the result of TOF/MS analysis of the antibody after the deglycosylation treatment (glycan removed antibody) is shown in Fig. 2B, respectively. In addition, each peak position (m/z) and a signal-to-noise ratio (S/N) thereof are shown in Table 1 given below. The schematic diagrams of the basic structures of the N-linked glycan and the partially removed glycan corresponding to each peak are as shown in the above-described Fig. 1A and Fig. 1B.

**[Table 1]**

| | *m*/*z* | | S/N | |
|---|---|---|---|---|
| | Untreated antibody | Glycan removed antibody | Untreated antibody | Glycan removed antibody |
| Peptide fragment | - | 1158.555 | - | 1605 |
| Peptide fragment +G0F' | 2236.269 | 2236.302 | 121 | 93 |
| Peptide fragment +G0F" | 2399.019 | - | 29 | - |
| Peptide fragment +G0F | 2602.090 | - | 153 | - |
| Peptide fragment +G1F | 2764.150 | - | 303 | - |
| Peptide fragment +G2F | 2926.203 | - | 68 | - |

As shown in Table 1, Fig. 2A, and Fig. 2B, in the glycan removed antibody (antibody composition), peaks at 2399.019 m/z (G0F''), 2602.090 m/z (G0F), 2764.150 m/z (G1F), and 2926.203 m/z (G2F) which were observed in the untreated antibody (antibody composition) were not observed. In addition, in the glycan removed antibody (antibody composition), also observed was a decrease in peak corresponding to 2236.269 m/z (G0F') which was observed in the untreated antibody (antibody composition). Moreover, in the glycan removed antibody (antibody composition), the peak at 1158.555 m/z, which was not observed in the untreated antibody (antibody composition), was observed, which was confirmed as peaks of the peptide fragments obtained by removing the above-described N-linked glycans (G0F', G0F", G0F, G1F, G2F) in the Fc region of the mouse anti-human CRP monoclonal antibody. From this, it was confirmed that glycan entirely removed antibodies in which at least the N-linked glycans of G0F', G0F", G0F, G1F, and G2F in the Fc region were removed from the mouse anti-human CRP monoclonal antibody by the deglycosylation treatment of (1) (cleaved and removed by glycopeptidase F) were obtained. Since in the mouse anti-human CRP monoclonal antibody (IgG1), a consensus sequence (Asn-Ser-Thr) was present only at positions 297 to 299 based on the Kabat numbering in the Fc region, it was confirmed that the N-linked glycan at position 297 in the antibody was removed.

In addition, from the result of the glycan analysis of the antibodies, the obtained glycan removed antibody (antibody composition) had such a significantly high homogeneity that the relative priority of the glycan entirely removed antibody (Pi: the amount of the glycan entirely removed antibody/the total antibody amount) was 0.89 and the diversity index (Simpson's diversity index) was 0.1.

### (3) Supporting of antibody on latex particle carrier

The glycan removed antibody (Example 1) prepared in (1) and polystyrene latex were mixed by a publicly-known method to support the glycan removed antibody on the latex surface (an obtained prepared solution comprising latex particles supporting the glycan removed antibody is referred to as a "glycan removed antibody-supporting latex emulsion"). In addition, as a comparison subject, the untreated antibody (Comparative Example 1) was treated in the same manner to prepare latex particles supporting the untreated antibody (an obtained prepared solution comprising latex particles supporting the untreated antibody is referred to as an "untreated antibody-supporting latex emulsion").

### (4) Reactivity of measurement system utilizing latex particles supporting glycan removed antibody or untreated antibody

For each of the glycan removed antibody-supporting latex emulsion and the untreated antibody-supporting latex emulsion prepared in the above (3), the reactivity with CRP was compared. First, as a first reagent, a reagent comprising a Good's buffer and a reaction modifier was prepared. As a second reagent, a reagent comprising a Good's buffer, a reaction modifier, and each antibody-supporting latex emulsion was prepared. In addition, antigen samples were prepared by diluting a CRP antigen to concentrations of 0.5, 2, 4, 20, 40, 50, and 100 mg/dL.

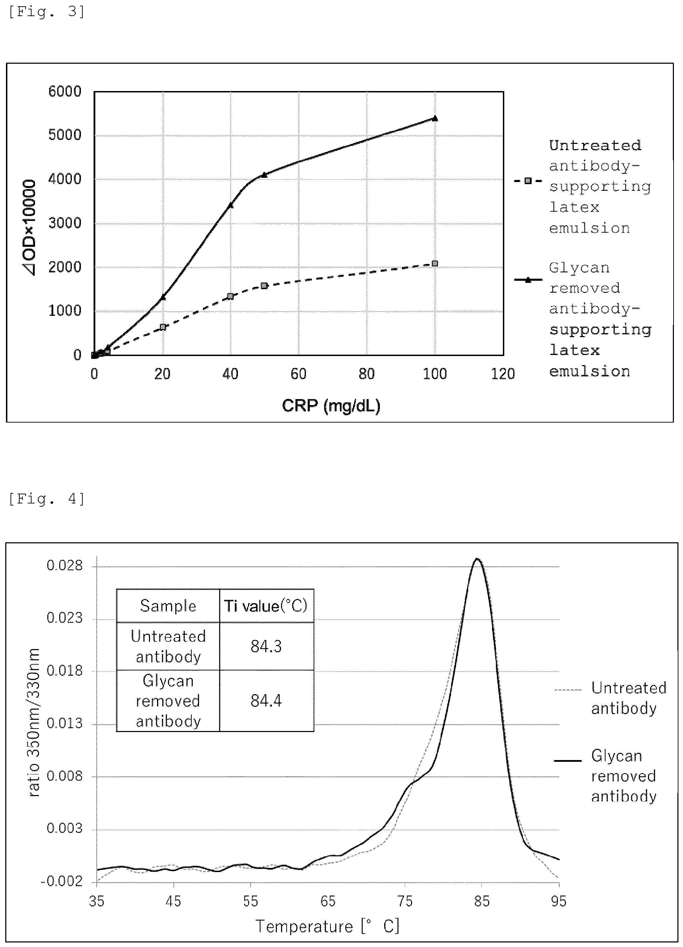

Subsequently, to 80 µL of the first reagent, an equal amount of the second reagent and 1.6 µL of antigen samples prepared at various concentrations were added, and the reaction was measured by an agglutination method, that is, as OD values by using automatic analyzer 7180 (manufactured by Hitachi High-Tech) (main wavelength: 570 nm, sub wavelength: 800 nm). From the OD value measured using the glycan removed antibody-supporting latex emulsion or the untreated antibody-supporting latex emulsion at each antigen sample concentration, a ΔOD value was calculated in accordance with the following formula: the ΔOD value=the OD value at a wavelength of 570 nm-the OD value at a wavelength of 800 nm. The relations between the obtained ΔOD values (ΔOD×10000) and the antigen sample concentrations are shown in Table 2 given below and Fig. 3. In addition, Table 2 also shows the ratio of the ΔOD value (glycan removed antibody ΔOD) (glycan removed antibody ΔOD/untreated antibody ΔOD×100, OD value ratio (%)) measured and calculated by using the glycan removed antibody-supporting latex emulsion when the ΔOD value (untreated antibody ΔOD) measured and calculated by using the untreated antibody-supporting latex emulsion is considered as 100%.

**[Table 2]**

| Antigen sample (mg/dL) | Untreated antibody-supporting latex emulsion (ΔOD×10000) | Glycan removed antibody-supporting latex emulsion (ΔOD×10000) | OD value ratio |
|---|---|---|---|
| 0 | 5 | 6 | - |
| 0.5 | 13 | 21 | 162% |
| 2 | 55 | 90 | 163% |
| 4 | 105 | 176 | 167% |
| 20 | 632 | 1322 | 209% |
| 40 | 1336 | 3430 | 257% |
| 50 | 1580 | 4108 | 260% |
| 100 | 2081 | 5395 | 259% |

As shown in Table 2 and Fig. 3, with 40 mg/dL or more of the antigen sample, for example, the ΔOD value, that is, the reactivity (the binding capability with the antigen) was improved about 2.5 times or more when the glycan removed antibody-supporting latex emulsion was used as compared with when the untreated antibody-supporting latex emulsion was used.

### (Test Example 2) Analysis of properties of glycan removed antibody

### (1) Confirmation of thermal Stabilities

The glycan removed antibody prepared in (1) of Test Example 1 was diluted with 10 mM HEPES to 1 mg/mL to prepare a sample, and the thermal denaturation curve was obtained and the inflection-point temperature (Ti) was determined by using Tycho NT.6 (manufactured by M&S TechnoSystems, Inc., wavelengths: 350 nm, 330 nm). In addition, as a comparison subject, the untreated antibody was treated in the same manner and was subjected to Tycho NT.6.

The thermal denaturation curves (the vertical axis: fluorescence intensity ratio between 2 wavelengths (ratio 350 nm/330 nm)) and the Ti values of the glycan removed antibody and the untreated antibody are as shown in Fig. 4. Since the difference in inflection-point temperature (Ti) between the glycan removed antibody and the untreated antibody was 0.1°C, it was confirmed that the deglycosylation treatment of (1) of Test Example 1 did not affect the thermal stability of an antibody.

### (2) Confirmation of binding forces of glycan removed antibodies to antigen by using Biacore

The binding amounts (affinities) of the glycan removed antibodies with an antigen were measured by using surface plasmon resonance analyzer Biacore T200 (manufactured by GE Healthcare). First, as antibody samples, the glycan removed antibody prepared in (1) of Test Example 1 and a glycan removed antibody exposed to reaction conditions (however, under a condition in which the latex solution was not added in the conditions of (3) of Test Example 1, hereinafter referred to as a "latex support condition") for being supported on latex particles in (3) of Test Example 1 were prepared. In addition, as comparison subjects, the above-described untreated antibody and an untreated antibody exposed to the above-described latex support condition were subjected to the test. In addition, as antigen samples, a dilution series was prepared by serially diluting a CRP antigen twice from 100 nM to 6.3 nM in HBS-P buffer (produced by GE Healthcare) to prepare CRP antigen solutions. Subsequently, a diluted solution obtained by diluting a 1 mg/mL anti-mouse IgG antibody comprised in a Mouse antibody capture kit (produced by GE Healthcare) to 30 ug/mL with 10 mM sodium acetate (pH 5.0) was supplied to a flow path of a sensor chip CM5 (produced by GE Healthcare) which was coated with carboxymethyl dextran at a flow speed of 10 µL/min for 7 minutes to be fixed by the amine coupling method.

To the sensor chip prepared as described above, first, HBS-P buffer was put into the flow path at a flow speed of 30 µL/min at 25°C. Subsequently, a solution of the antibody sample diluted to 10 ug/mL with HBS-P buffer was put into the flow path for 15 seconds to be coupled to the anti-mouse IgG antibody fixed on the sensor chip. Thereafter, the CRP antigen solution was put into the flow path for 120 second to observe binding reaction, and then the HBS-P buffer was put into the flow path for 180 seconds to observe dissociation reaction. Based on sensorgrams thus obtained, the binding amount of each antibody and the antigen was calculated by using analysis software attached to the device.

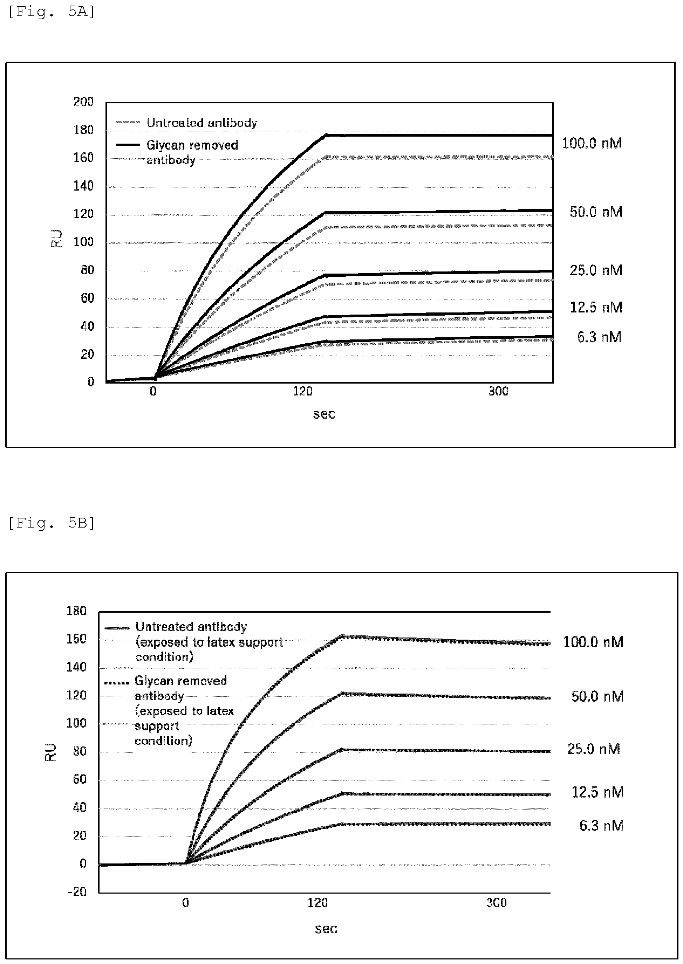

The binding amount (CRP (RU)) of the antigen when each antibody sample was used for the antigen solution at each concentration is shown in Table 3 given below, Fig. 5A, and Fig. 5B. Table 3 also shows the binding amount of each antibody (mAb (RU)), the ratio of the binding amount of the antigen when the binding amount of each antibody was considered as 100% (CRP (RU)/mAb (RU)×100, CRP/mAb (%)), and the ratio of CRP/mAb when the glycan removed antibody was used when CRP/mAb in using the untreated antibody was considered as 100% (CRP/mAb value ratio (%)). Note that since each % value shown in Table 3 was such that the actual value calculated from each measured value is represented by significant figures shown in Table, the % value does not necessarily coincide with the % value calculated from the values shown in Table (the same applies below).

**[Table 3]**

| Antigen solution concentration | Antibody sample | mAb (RU) | CRP (RU) | CRP/mAb | CRP/mAb value ratio |
|---|---|---|---|---|---|
| 6.3 nM | Untreated antibody | 317.5 | 29.1 | 9.2% | 100.9% |
| | Glycan removed antibody | 339.5 | 31.4 | 9.2% | |
| | Untreated antibody (exposed to the latex support condition) | 311.2 | 32.7 | 10.5% | 104.0% |
| | Glycan removed antibody (exposed to the latex support condition) | 300.2 | 32.8 | 10.9% | |
| 12.5 nM | Untreated antibody | 313.5 | 44.8 | 14.3% | 100.5% |
| | Glycan removed antibody | 339.0 | 48.7 | 14.4% | |
| | Untreated antibody (exposed to the latex support condition) | 309.6 | 53.2 | 17.2% | 103.8% |
| | Glycan removed antibody (exposed to the latex support condition) | 299.4 | 53.4 | 17.8% | |
| 25 nM | Untreated antibody | 310.8 | 71.1 | 22.9% | 100.4% |
| | Glycan removed antibody | 337.1 | 77.4 | 23.0% | |
| | Untreated antibody (exposed to the latex support condition) | 308.4 | 84.2 | 27.3% | 103.5% |
| | Glycan removed antibody (exposed to the latex support condition) | 298.8 | 84.4 | 28.2% | |
| 50 nM | Untreated antibody | 308.9 | 110.9 | 35.9% | 100.0% |
| | Glycan removed antibody | 336.9 | 120.9 | 35.9% | |
| | Untreated antibody (exposed to the latex support condition) | 308.0 | 124.1 | 40.3% | 103.1% |
| | Glycan removed antibody (exposed to the latex support condition) | 298.5 | 124.0 | 41.5% | |
| 100 nM | Untreated antibody | 306.2 | 160.9 | 52.5% | 99.3% |
| | Glycan removed antibody | 336.5 | 175.5 | 52.2% | |
| | Untreated antibody (exposed to the latex support condition) | 306.9 | 164.8 | 53.7% | 102.5% |
| | Glycan removed antibody (exposed to the latex support condition) | 298.0 | 164.0 | 55.0% | |
| Average of CRP/mAb value ratios | Glycan removed antibody/Untreated antibody | | | | 100.2% |
| | Glycan removed antibody (exposed to the latex support condition)/Untreated antibody (exposed to the latex support condition) | | | | 103.4% |

As shown in Table 3, Fig. 5A, and Fig. 5B, the CRP/mAb values were 0.2% higher on average when the glycan removed antibodies were used than when the untreated antibodies were used. In addition, the CRP/mAb values were 3.4% higher on average when glycan removed antibodies exposed to the latex support condition were used than when the untreated antibodies exposed to the latex support condition were used.

### (3) Confirmation of binding forces of glycan removed antibodies Supported on latex particles to antigen by using Biacore

The binding amounts (affinities) of the glycan removed antibodies supported on latex particles and an antigen were measured by using surface plasmon resonance analyzer Biacore T200 (manufactured by GE Healthcare). First, as antibody samples, glycan removed antibody-supporting latex emulsions prepared in (3) of Test Example 1 were used. In addition, as comparison subjects, the untreated antibody-supporting latex emulsions prepared in (3) of Test Example 1 were subjected to the test. In addition, as antigen samples, a dilution series was prepared by serially diluting a CRP antigen twice from 1000 nM to 62.5 nM in HBS-P buffer to prepare CRP antigen solutions. The sensor chip was prepared in the same manner as in (2) of Test Example 2.

To the prepared sensor chip, first, HBS-P buffer was put into the flow path at a flow speed of 30 µL/min at 25°C. Subsequently, a solution of each antibody sample diluted 37 times with HBS-P buffer (equivalent to 10 µg/mL in terms of the antibody amount) was put into the flow path for 15 seconds to be bound to the anti-mouse IgG antibody fixed on the sensor chip, and then the CRP antigen solution was put into the flow path for 120 seconds to observe the binding reaction. Thereafter, HBS-P buffer was put into the flow path for 180 seconds to observe the dissociation reaction. Based on sensorgrams thus obtained, the binding amount of each antibody supported on the latex particles and the antigen was calculated by using analysis software attached to the device.

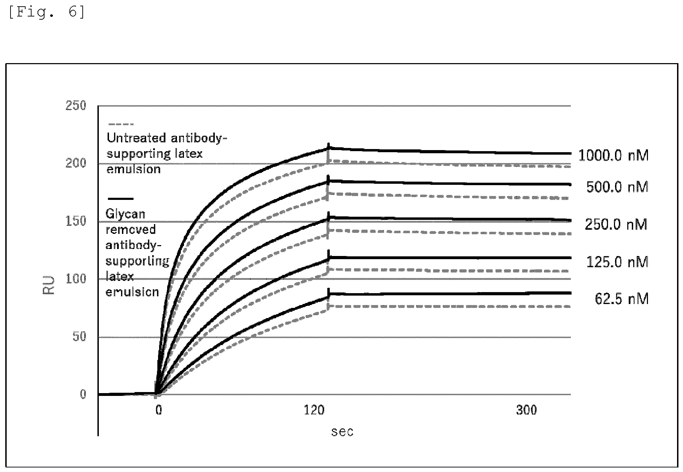

The binding amount (CRP (RU)) of the antigen when each antibody sample was used for the antigen solution at each concentration is shown in Table 4 given below and Fig. 6. In addition, Table 4 also shows the binding amount of each antibody supported on the latex particles (latex (RU)), the ratio of the binding amount of the antigen when the binding amount of each antibody was considered as 100% (CRP(RU)/latex(RU)×100, CRP/latex(%)), and the ratio of CRP/latex when the glycan removed antibody-supporting latex emulsion was used when the CRP/latex in using the untreated antibody-supporting latex emulsion was considered as 100% (CRP/latex value ratio (%)).

**[Table 4]**

| Antigen solution concentration | Antibody sample | latex (RU) | CRP (RU) | CRP/Latex | CRP/Latex value ratio |
|---|---|---|---|---|---|
| 62.5 nM | Untreated antibody-supporting latex emulsion | 366.6 | 74.5 | 20.3% | 116.4% |
| | Glycan removed antibody-supporting latex emulsion | 349.2 | 82.6 | 23.7% | |
| 125.0 nM | Untreated antibody-supporting latex emulsion | 378.8 | 100.0 | 26.4% | 120.6% |
| | Glycan removed antibody-supporting latex emulsion | 349.9 | 111.4 | 31.8% | |
| 250.0 nM | Untreated antibody-supporting latex emulsion | 374.6 | 133.9 | 35.7% | 117.1% |
| | Glycan removed antibody-supporting latex emulsion | 350.5 | 146.7 | 41.9% | |
| 500.0 nM | Untreated antibody-supporting latex emulsion | 371.6 | 166.4 | 44.8% | 114.4% |
| | Glycan removed antibody-supporting latex emulsion | 349.9 | 179.2 | 51.2% | |
| 1000.0 nM | Untreated antibody-supporting latex emulsion | 370.5 | 195.9 | 52.9% | 112.5% |
| | Glycan removed antibody-supporting latex emulsion | 349.6 | 207.9 | 59.5% | |
| Average of CRP/Latex value ratios | | | | | 116.2% |

As shown in Table 4 and Fig. 6, also in immobilized antibodies, it was confirmed that the CRP/latex values were higher when the glycan removed antibodies were used, that is, when the glycan removed antibody-supporting latex emulsions were used than when the untreated antibodies were used, that is, the untreated antibody-supporting latex emulsions were used, and that the reactivities (binding forces) with the antigen were improved. In addition, the CRP/latex value was 16.2% higher on average, and it was confirmed that the improvement in reactivity of the antibody with the antigen due to the deglycosylation was exerted particularly when the antibody was supported on a carrier.

### (Test Example 3) Evaluation of reactivity of anti-human Hb monoclonal antibody (enzyme-treated antibody) from which glycan was removed by using enzyme (glycopeptidase F treatment)

### (1) Preparation of antibodies from which glycan was removed

To 1 mg of each of an anti-human Hb (hemoglobin) monoclonal antibody A (IgG1, hereinafter referred to as an "antibody A") and an anti-human Hb monoclonal antibody B (IgG1, hereinafter referred to as an "antibody B") different from the antibody A, 1 µL of glycopeptidase F with 500 µU/µL (produced by Takara Bio Inc.) was added, followed by keeping at 37°C for 18 hours to prepare an antibody A subjected to the deglycosylation treatment (hereinafter referred to as a "glycan removed antibody A") and an antibody B subjected to the deglycosylation treatment (hereinafter referred to as a "glycan removed antibody B").

### (2) Glycan analysis of antibodies

First, to 10 µg of each glycan removed antibody prepared in (1), 10 µL of 8 M Urea-20 mM ammonium bicarbonate (pH 8.0) and 1 µL of 100 mM DTT were added, followed by keeping at 70°C for 3 minutes. Subsequently, 90 µL of 20 mM ammonium bicarbonate (pH 8.0) and 1 µL of 500 µg/mL Trypsin solution were added, followed by keeping at 37°C for 5 minutes, and further 1 µL of 10% trifluoroacetic acid was added to obtain a prepared solution. The prepared solution thus obtained was put into C18 ziptip (produced by Merck KGaA), and was eluted with 9% acetonitrile/0.1% trifluoroacetic acid. To the eluate thus obtained, an equal amount of HCCA (produced by Bruker) saturated using 100% acetonitrile was added, followed by mixing to prepare a sample solution. The sample solution thus obtained was subjected to MALDI-TOF/MS (produced by Bruker). In addition, as comparison subjects, an antibody A (hereinafter referred to as an "untreated antibody A") and an antibody B (hereinafter referred to as an "untreated antibody B") which were not subjected to the deglycosylation treatment of (1) were treated in the same manner and were subjected to MALDI-TOF/MS.

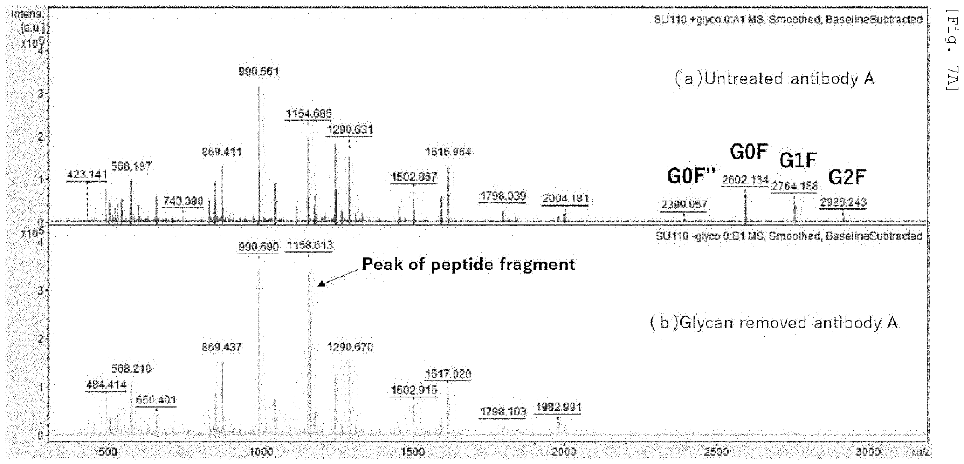

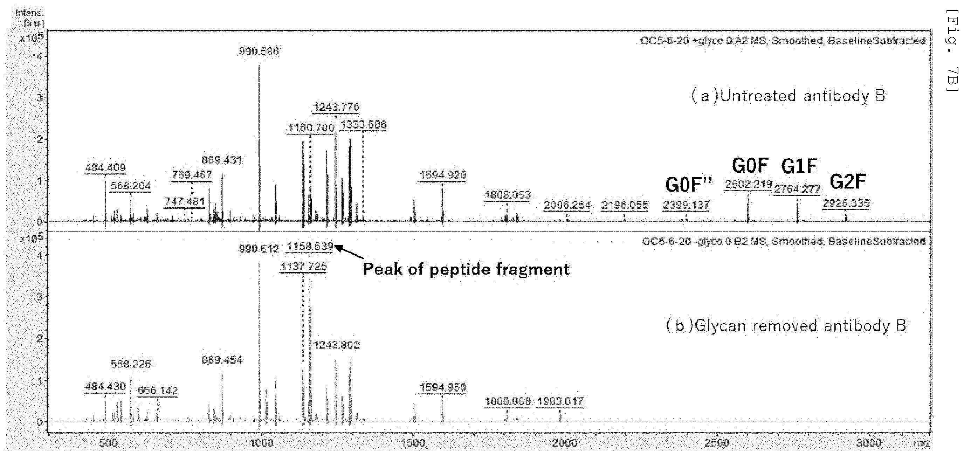

The result of the TOF/MS analysis of the antibodies (the untreated antibodies A, B) before the deglycosylation treatment is shown in (a) of Fig. 7A and Fig. 7B, and the result of the TOF/MS analysis of the antibodies (the glycan removed antibodies A, B) after the deglycosylation treatment is shown in (b) of Fig. 7A and Fig. 7B. In addition, each peak position (m/z) and a signal-to-noise ratio (S/N) thereof are shown in Table 5 given below. The schematic diagrams of the basic structures of the N-linked glycan and the partially removed glycan corresponding to each peak are as shown in the above-described Fig. 1A and Fig. 1B.

**[Table 5]**

| | | m/z | | S/N | |
|---|---|---|---|---|---|
| | | Untreated antibody | Glycan removed antibody | Untreated antibody | Glycan removed antibody |
| Antibody A | Peptide fragment | - | 1158.613 | - | 1100 |
| | Peptide fragment+GOF" | 2399.057 | - | 87 | - |
| | Peptide fragment+G0F | 2602.134 | - | 1353 | - |
| | Peptide fragment+G1F | 2764.188 | - | 1246 | - |
| | Peptide fragment+G2F | 2926.243 | - | 298 | - |
| Antibody B | Peptide fragment | - | 1158.639 | - | 1539 |
| | Peptide fragment+G0F" | 2399.137 | - | 91 | - |
| | Peptide fragment+G0F | 2602.219 | - | 1281 | - |
| | Peptide fragment+G1F | 2764.277 | - | 1252 | - |
| | Peptide fragment+G2F | 2926.335 | - | 346 | - |

As shown in Table 5, Fig. 7A, and Fig. 7B, in both of the antibody A and the antibody B, peaks of G0F", G0F, G1F, and G2F were observed in the untreated antibodies (antibody compositions), but were not observed in the glycan removed antibodies (antibody compositions), and instead, peaks of the peptide fragments obtained by removing the above-described N-linked glycans from the Fc regions of the antibody A and the antibody B were observed. From this, it was confirmed that glycan entirely removed antibodies in which at least the N-linked glycans of G0F", G0F, G1F, and G2F from the Fc regions of the antibody A and the antibody B were removed by the deglycosylation treatment of (1) (cleaved and removed by glycopeptidase F) were obtained. Since in the antibody A and the antibody B (both are IgG1), a consensus sequence (Asn-Ser-Thr) was present only at positions 297 to 299 based on the Kabat numbering in the Fc regions, it was confirmed that the N-linked glycan at position 297 in each of the antibody A and the antibody B was removed.

### (3) Supporting of antibodies on latex particle carrier

By a publicly-known method, the glycan removed antibody A (Example 2) or the glycan removed antibody B (Example 3) prepared in (1) and polystyrene latex were mixed to support each glycan removed antibody on the latex surface (a prepared solution comprising the obtained latex particles supporting the glycan removed antibody A (or B) is referred to as a "glycan removed antibody A (or B)-supporting latex emulsion"). In addition, as comparison subjects, the untreated antibody A (Comparative Example 2) and the untreated antibody B (Comparative Example 3) were treated in the same manner to prepare latex particles supporting the untreated antibodies (an obtained prepared solution comprising the latex particles supporting the untreated antibody A (or B) is referred to as an "untreated antibody A (or B)-supporting latex emulsion").

### (4) Reactivity of measurement system utilizing latex particles supporting glycan removed antibody or untreated antibody

For a mixture liquid I of the untreated antibody A-supporting latex emulsion and the untreated antibody B-supporting latex emulsion, a mixture liquid II of the glycan removed antibody A-supporting latex emulsion and the untreated antibody B-supporting latex emulsion, and a mixture liquid III of the untreated antibody A-supporting latex emulsion and the glycan removed antibody B-supporting latex emulsion, which were prepared in the above (3), the reactivity with Hb was compared. First, as a first reagent, a reagent comprising a Good's buffer and a reaction modifier was prepared. As a second reagent, a reagent comprising a Good's buffer, a reaction modifier, and each mixture liquid was prepared. In addition, antigen samples were prepared by diluting human hemoglobin (hHb) purified from human bloods to concentrations of 100, 250, 500, 750, and 1000 ng/mL.

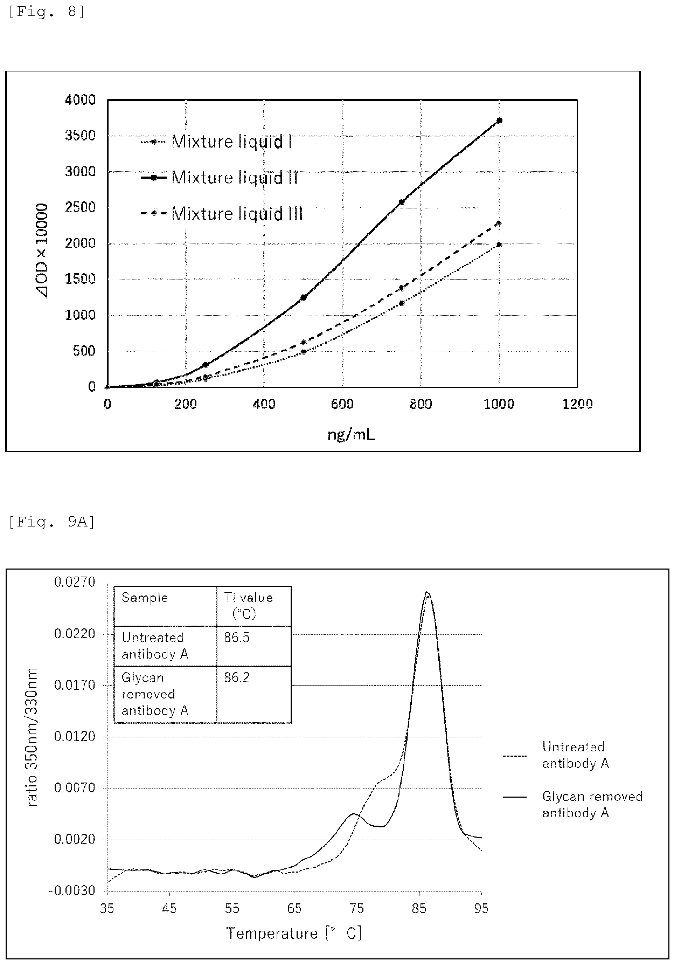

Subsequently, to 50 µL of the first reagent, 25 µL of the second reagent and 5.0 µL of the antigen samples prepared at various concentrations were added, and the reaction was measured by an agglutination method, that is, as OD values by using automatic analyzer JCA-BM6070 (manufactured by JEOL Ltd.) at measurement wavelength: 658 nm to obtain ΔOD values. The relations between the obtained ΔOD values (ΔOD×10000) and the antigen sample concentrations (ng/mL) are shown in Table 6 given below and Fig. 8. In addition, Table 6 also shows the ratio of the ΔOD value (glycan removed antibody ΔOD) (glycan removed antibody ΔOD/untreated antibody ΔOD×100, OD value ratio (%)) measured by using the mixture liquid II or III when the ΔOD value (untreated antibody ΔOD) measured by using the mixture liquid I is considered as 100%.

**[Table 6]**

| Antigen sample (ng/mL) | Mixture liquid I (ΔOD×10000) | Mixture liquid II (ΔOD×10000) | OD value ratio | Mixture liquid III (ΔOD×10000) | OD value ratio |
|---|---|---|---|---|---|
| 0 | 1 | 2 | | 2 | |
| 100 | 32 | 67 | 207% | 38 | 117% |
| 250 | 121 | 308 | 254% | 151 | 124% |
| 500 | 493 | 1252 | 254% | 626 | 127% |
| 750 | 1176 | 2579 | 219% | 1383 | 118% |
| 1000 | 1988 | 3719 | 187% | 2295 | 115% |

As shown in Table 6 and Fig. 8, with 250 to 500 ng/mL of the antigen sample, for example, the ΔOD value, that is, the reactivity (the binding capability with the antigen) was improved about 2.5 times or more when glycans were removed only from the antibody A (the mixture liquid II), and about 1.2 times or more when glycans were removed only from the antibody B (the mixture liquid III), as compared with when the mixture liquid I of the untreated antibody-supporting latex emulsion was used.

### (Test Example 4) Analysis of properties of glycan removed antibody

### (1) Confirmation of thermal stabilities

The glycan removed antibodies A and B prepared in (1) of Test Example 3 were each diluted with 10 mM HEPES to 1 mg/mL to prepare samples, and the thermal denaturation curves were obtained and the inflection-point temperatures (Ti) were determined by using Tycho NT.6 (manufactured by M&S TechnoSystems, Inc., wavelengths: 350 nm, 330 nm). In addition, as comparison subjects, the untreated antibodies A and B were each treated in the same manner and were subjected to Tycho NT.6.

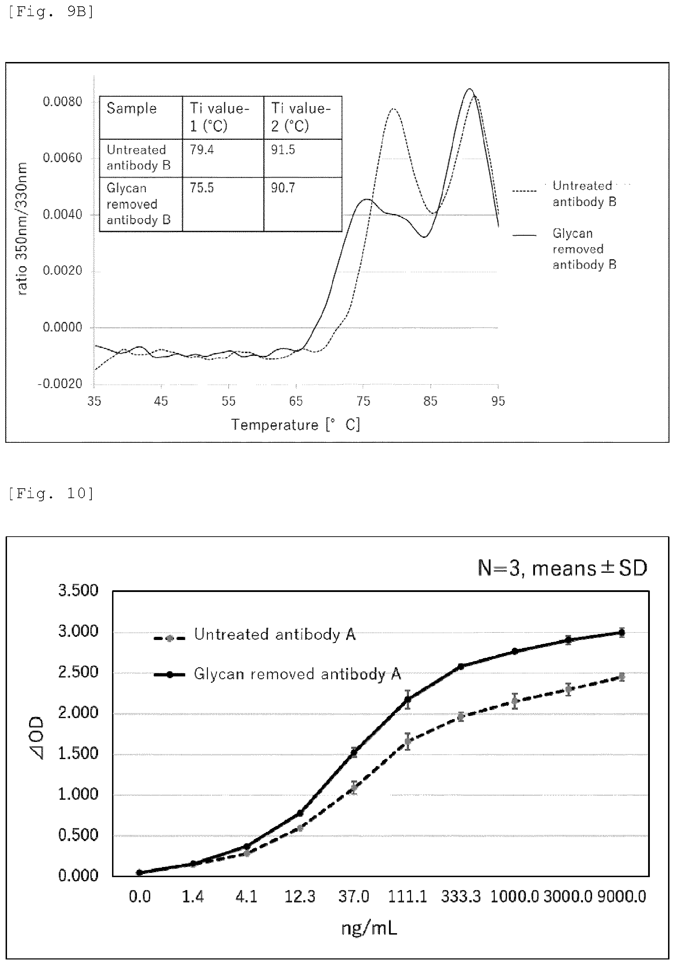

The thermal denaturation curves (the vertical axis: fluorescence intensity ratio between 2 wavelengths (ratio 350 nm/330 nm)) and the Ti values of the glycan removed antibody A and the untreated antibody A are as shown in Fig. 9A, and the thermal denaturation curves and the Ti values of the glycan removed antibody B and the untreated antibody B are as shown in Fig. 9B. In either of the antibody A and the antibody B, there was hardly a difference in inflection-point temperature (Ti) between the glycan removed antibody and the untreated antibody, and the deglycosylation treatment of (1) of Test Example 3 did not affect the thermal stabilities of antibodies themselves. However, in Fig. 9A and Fig. 9B, two peaks corresponding to the CH2 region and the Fab region were observed in the antibody A and the antibody B, and it was confirmed that the thermal stability of the CH2 region decreased in the glycan removed antibodies (for example, while in the antibody B (Fig. 9B), a decrease in Ti value (Ti value-2) of the Fab region was 0.8°C, a decrease in Ti value (Ti value-1) of the CH2 region was 3.9°C).

### (2) Confirmation of binding forces of glycan removed antibodies to antigen using sandwich ELISA

First, as capturing bodies, each of the glycan removed antibody A and the untreated antibody A prepared in (1) of Test Example 3 was diluted with 50 mM carbonate buffer (pH 9.6) to 1 mg/mL, and was dispensed in 100 µL per well of a Nunc-Immuno Module, F8 (manufactured by Thermo Fisher Scientific Inc.) plate. After kept at room temperature for 1 hour, the wells were washed three times by using 350 µL of cleaning liquid 2, and a Blocking Buffer 2 was added in 250 µL per well, followed by keeping at room temperature for 1 hour to prepare an ELISA plate. In addition, antibody B-HRP labeled antibodies were obtained by directly labeling the glycan removed antibody B and the untreated antibody B prepared in (1) of Test Example 3 with HRP, and as labeled bodies, labeled body fluids were prepared by diluting the antibody B-HRP labeled antibodies 5,000 times with an antibody diluent (1% BSA in PBS-T). In addition, a human blood-derived purification Hb antigen solution was serially diluted three times from 9,000 ng/mL to 1.4 ng/mL with Blocking Buffer 2 to obtain an antigen solution.

Subsequently, the antigen solution was added in 100 µL per well in the prepared ELISA plate. After kept at room temperature for 1 hour, the wells were washed three times with 350 µL of PBS-T. Subsequently, the labeled body fluid was dispensed in 100 µL per well to obtain a glycan removed antibody B-HRP labeled antibody from the glycan removed antibody A and an untreated antibody B-HRP labeled antibody from the untreated antibody A. After kept at room temperature for 1 hour, the wells were washed three times with 350 µL of PBS-T. In a predetermined amount of a solvent consisting of a 0.1 M citric acid buffer solution (pH 5.5) and a 0.05% hydrogen peroxide solution, one tablet of an OPD tablet (produced by FUJIFILM Wako Pure Chemical Corporation) was dissolved to obtain a colored solution, which was added in 100 µL per well. After kept at room temperature for 15 minutes, a reaction stop solution (3N H₂SO₄) was added in 100 µL per well to stop the reaction. The absorbance at 492 nm and 650 nm was measured in the plate reader, and a value (ΔOD) was calculated by subtracting the absorbance at 650 nm from the absorbance at a wavelength of 492 nm. The ΔOD value obtained by using each antibody A as a capturing body at each antigen solution concentration (ng/mL) is shown in Table 7 given below and Fig. 10. Table 7 also shows the ratio of the ΔOD value (ΔOD value ratio (%)) measured by using the glycan removed antibody A as the capturing body when the ΔOD value measured by using the untreated antibody A as the capturing body is considered as 100%.

**[Table 7]**

| Antigen solution concentration (ng/mL) | Untreated antibody A (ΔOD) | Glycan removed antibody A (ΔOD) | ΔOD value ratio |
|---|---|---|---|
| 0.0 | 0.044 | 0.045 | 102% |
| 1.4 | 0.147 | 0.158 | 108% |
| 4.1 | 0.283 | 0.367 | 130% |
| 12.3 | 0.600 | 0.778 | 130% |
| 37.0 | 1.087 | 1.522 | 140% |
| 111.1 | 1.657 | 2.171 | 131% |
| 333.3 | 1.962 | 2.578 | 131% |
| 1000.0 | 2.151 | 2.761 | 128% |
| 3000.0 | 2.297 | 2.900 | 126% |
| 9000.0 | 2.448 | 2.994 | 122% |

As shown in Table 7 and Fig. 10, it was confirmed that in the sandwich ELISA using the glycan removed antibody A as the capturing body and the glycan removed antibody B as the labeled body, for example, at an antigen concentration of 37.0 ng/mL, the reactivity (binding force) to the antigen was improved 1.4 times as compared with the sandwich ELISA using the untreated antibody A as the capturing body and the untreated antibody B as the labeled body.

### (Test Example 5) Evaluation of reactivity of mouse anti-human CRP monoclonal antibodies from which glycans were recombined and removed

### (1) Preparation of antibodies (recombinant antibodies) from which glycans were recombined and removed

mRNA was extracted from hybridoma producing a monoclonal antibody exhibiting high reactivity to human CRP, to thus synthesize cDNA. Subsequently, with the obtained cDNA as a template, PCR was conducted by using N297S-primer F (SEQ ID NO: 1) and N297S-primer R (SEQ ID NO: 2) produced based on a typical sequence of a mouse anti-human CRP monoclonal antibody (IgG1) to obtain a DNA fragment encoding a recombinant antibody (N297S) in which asparagine (N) at position 297 based on the Kabat numbering of the above antibody was replaced with serine (S). In addition, PCR was conducted by using N297C-S298C-primer F (SEQ ID NO: 3) and N297C-S298C-primer R (SEQ ID NO: 4) to obtain a DNA fragment encoding a recombinant antibody (N297C-S298C) in which asparagine (N) at position 297 and serine (S) at position 298 based on the Kabat numbering of the above antibody were replaced with cysteine (C) and cysteine (C), respectively. Each DNA fragment thus obtained was inserted into pcDNA 3.1D/V5-His-TOPO (Thermo Fisher Scientific Inc.). Antibody expression was conducted in ExpiCHO Expression System (Thermo Fisher Scientific Inc.) by using the prepared vectors. The antibodies thus expressed were purified through Protein A purification and gel filtration purification to obtain recombinant antibodies N297S and N297C-S298C, respectively. In addition, as a comparison subject, PCR was conducted with the above cDNA as a template to obtain a DNA fragment encoding the above typical sequence. An antibody WT in which the above modification (amino acid replacement) was not introduced was obtained by using each DNA fragment thus obtained.

### (2) Glycan analysis of the antibodies

First, 10 µL of 8 M Urea-20 mM ammonium bicarbonate (pH 8.0) and 1 µL of 100 mM DTT were added to 10 µg of each of the recombinant antibodies (N297S (Example 4) and N297C-S298C (Example 5)) prepared in (1), followed by keeping at 70°C for 3 minutes. Subsequently, 90 µL of 20 mM ammonium bicarbonate (pH 8.0) and 1 µL of 500 ug/mL Trypsin solution were added, followed by keeping at 37°C for 5 minutes, and then, 1 µL of 10% trifluoroacetic acid was further added to obtain prepared solutions. The prepared solutions thus obtained were put into C18 ziptip (manufactured by Merck KGaA) and were eluted with 9% acetonitrile/0.1% trifluoroacetic acid. An equal amount of HCCA (produced by Bruker) saturated using 100% acetonitrile was added to and mixed with the eluates thus obtained to prepare sample solutions. The sample solutions thus obtained were subjected to MALDI-TOF/MS (produced by Bruker). In addition, WT (Comparative Example 4) prepared in (1) was treated in the same manner and was subjected to MALDI-TOF/MS.

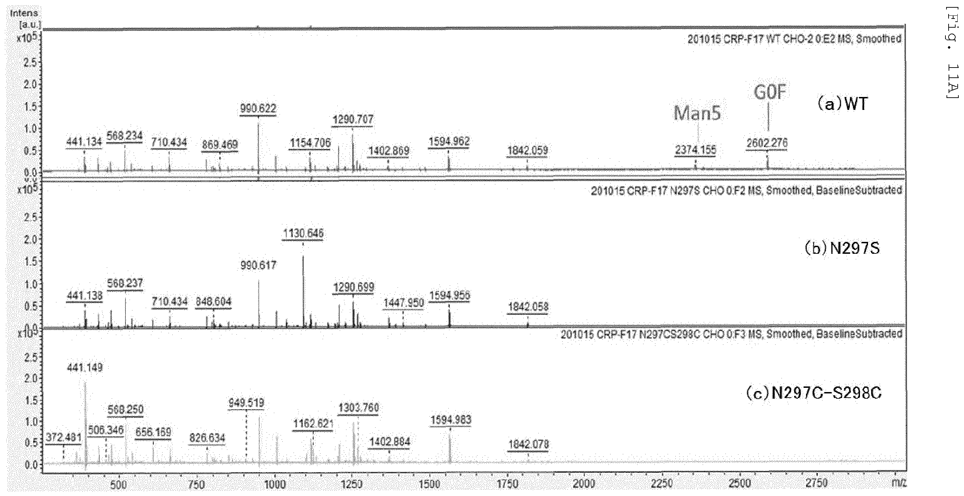

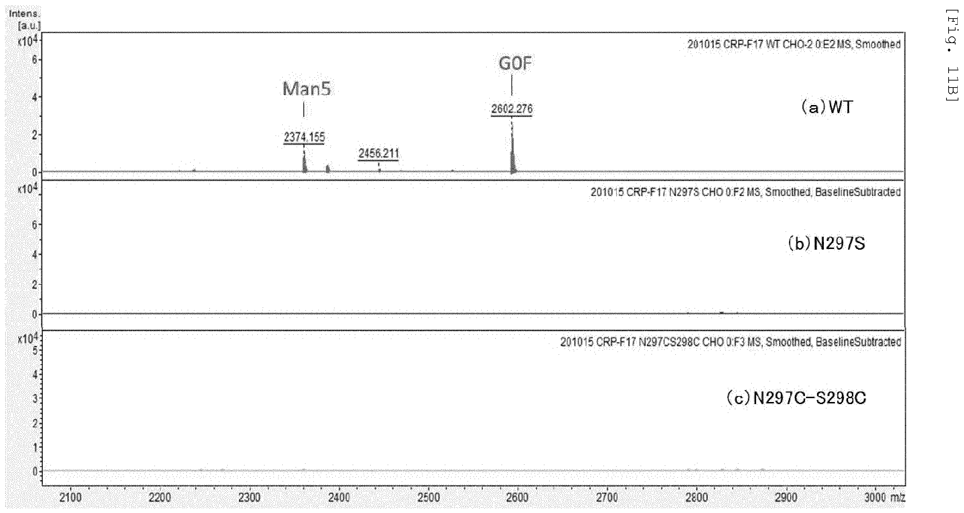

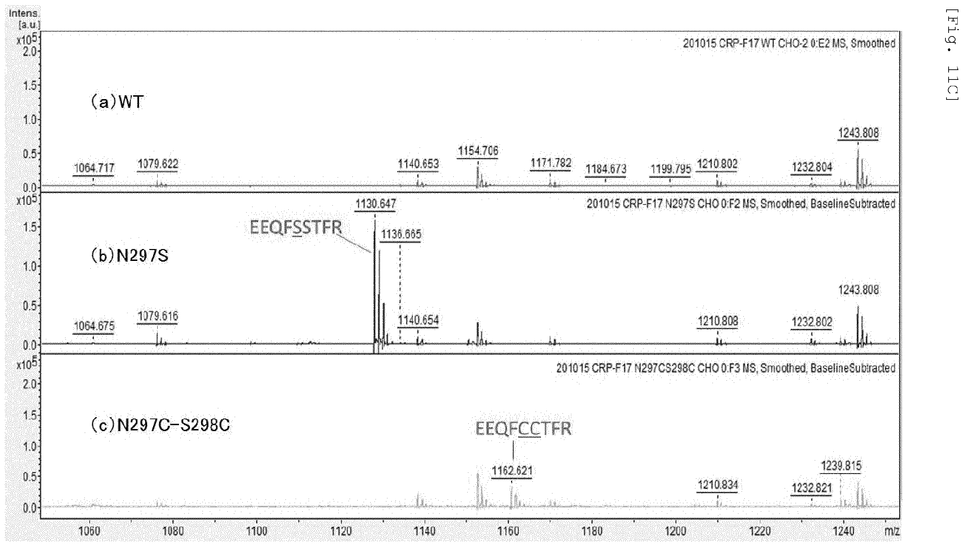

The result of TOF/MS analysis of the antibody (WT) in which asparagine at position 297 was not replaced is shown in (a) of Fig. 11A, Fig. 11B, and Fig. 11C, and the results of TOF/MS analysis of the antibodies (N297S, N297C-S298C) in which asparagine at position 297 was replaced are shown in (b), (c) of Fig. 11A, Fig. 11B, and Fig. 11C. Fig. 11B and Fig. 11C are enlarged diagrams of Fig. 11A. The schematic diagram of the basic structure of the N-linked glycan corresponding to each peak is as shown in the above-described Fig. 1A.

As shown in Fig. 11A, Fig. 11B, and Fig. 11C, peaks of N-linked glycans of Man5 and G0F were observed in WT (antibody composition), but were not observed in the recombinant antibodies (N297S and N297C-S298C, each of which was an antibody composition) (Fig. 11A, Fig. 11B), and instead, peaks of peptide fragments in which the N-linked glycans of the Fc region of the mouse anti-human CRP monoclonal antibodies were removed and position N297 was replaced with S ((b) of Fig. 11C) or both positions N297 and S298 were replaced with C ((c) of Fig. 11C) were observed. From this, it was confirmed that glycan entirely removed antibodies in which N-linked glycans of Man5 and G0F were removed from position 297 of the Fc region of the mouse anti-human CRP monoclonal antibodies by the replacement of asparagine at position 297 based on the Kabat numbering in (1) were obtained.

### (3) Confirmation of thermal stabilities

The antibodies in which glycans were removed (N297S and N297C-S298C) and the antibody in which glycan was not removed (WT), which were prepared in the above-described (1), were each diluted with 10 mM HEPES to 1 mg/mL to prepare samples, and the thermal denaturation curves were obtained and the inflection-point temperatures (Ti) were determined by using Tycho NT.6 (manufactured by M&S TechnoSystems, Inc., wavelengths: 350 nm, 330 nm). In addition, as a reference, the glycan removed antibody (in Test Example 5, referred to as a "Hybridoma glycan removed antibody") and the untreated antibody (in Test Example 5, referred to as a "Hybridoma-untreated antibody"), which were prepared in (1) of Test Example 1, were treated in the same manner and were subjected to Tycho NT.6.

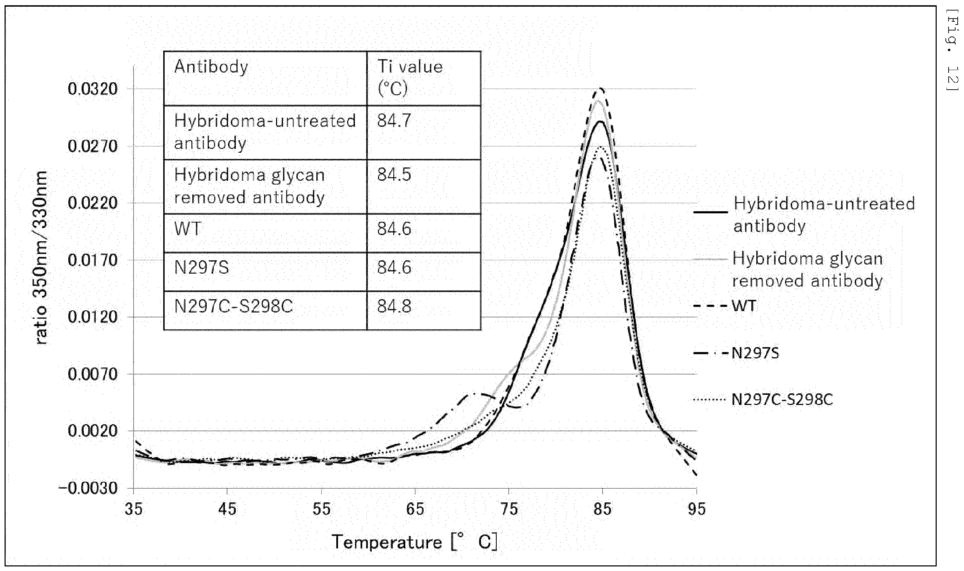

The thermal denaturation curves (the vertical axis: the fluorescence intensity ratio between 2 wavelengths (ratio 350 nm/330 nm)) and the Ti values of the recombinant antibodies (N297S and N297C-S298C), WT, as well as the Hybridoma glycan removed antibody and the untreated antibody are shown in Fig. 12. There was hardly a difference in inflection-point temperature (Ti) among the antibodies, and it was confirmed that the deglycosylation treatment (replacement at position N297) of (1) did not affect the thermal stabilities of antibodies themselves. However, in Fig. 12, two peaks of the CH2 region and the Fab region were observed in N297S and the Hybridoma glycan removed antibody, particularly in N297S, and it was confirmed that the thermal stability of the CH2 region decreased due to the removal of glycans. In contrast, such peaks were not observed in N297C-S298C, and it was confirmed that the thermal stability of the CH2 region was maintained even when glycan was removed.

### (4) Confirmation of disulfide bond

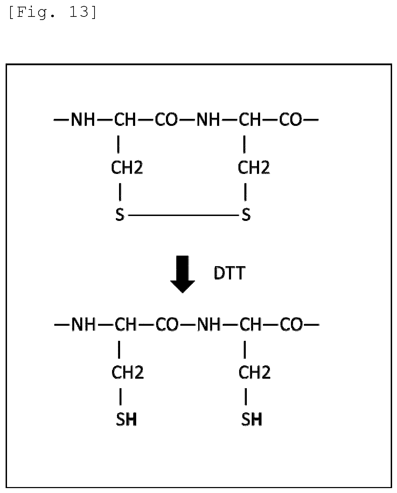

From the above-described (3), it was considered that the maintenance of the thermal stability of the CH2 region in N297C-S298C was caused by formation of a disulfide bond between adjacent cysteines. Hence, it was checked whether or not cleavage of the disulfide bond shown in Fig. 13 was caused by reduction using DTT (dithiothreitol) in a method described below. That is, in the case where protons were added by reduction using DTT, the molecular weight increases by that (2Da), the presence of a disulfide bond can be checked by checking an increase in molecular weight due to the reduction.

First, 10 µL of 8 M Urea-20 mM ammonium bicarbonate (pH 8.0) and 1 µL of 100 mM DTT were added to each of the recombinant antibodies (N297S and N297C-S298C) prepared in (1), followed by keeping at 70°C for 3 minutes to conduct DTT reduction treatment. Then, peptides were fragmented by trypsin treatment to prepare sample solutions. The sample solutions thus obtained were subjected to MALDI-TOF/MS (produced by Bruker). In addition, as comparison subjects, N297S and N297C-S298C that were not subjected to DTT reduction treatment (that is, the same treatment as (2) except that 1 µL of 100 mM DTT was not added, non-reduction) were treated in the same manner and were subjected to MALDI-TOF/MS.

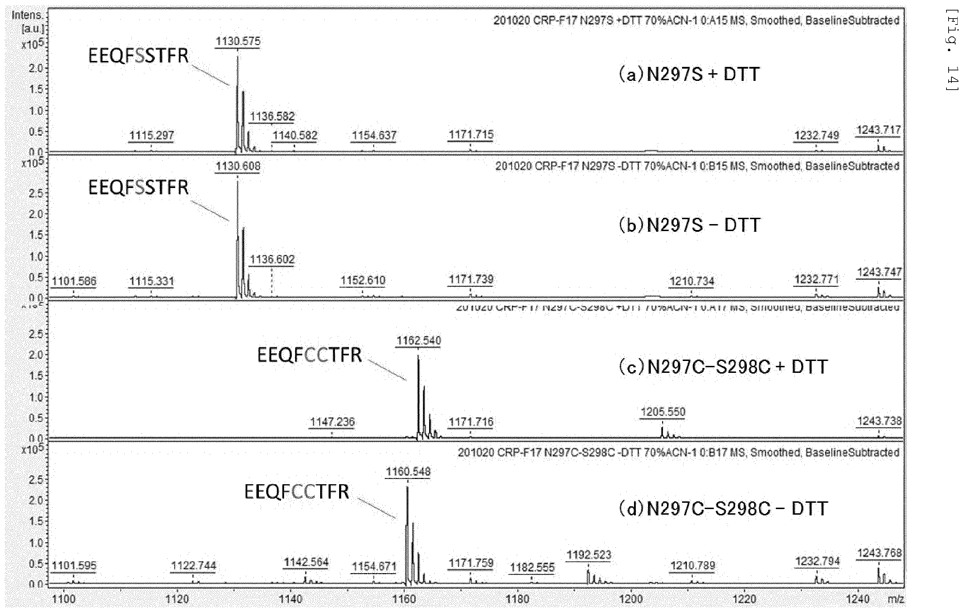

The result of TOF/MS analysis of N297S and N297C-S298C (+DTT) that were subjected to reduction treatment is shown in (a) and (c) of Fig. 14, and the result of TOF/MS analysis of non-reduced N297S and N297C-S298C (-DTT) is shown in (b) and (d) of Fig. 14. As shown in Fig. 14, while no change in peak was observed between reduced and non-reduced N297S, the peak of the peptide fragment was shifted by 2Da in N297C-S298C (c) that were subjected to the reduction treatment as compared with non-reduced N297C-S298C (d), so that it was confirmed that a disulfide bond was present in N297C-S298C. It can be said that this was formed between cysteines introduced to position 297 and position 298 based on the Kabat numbering.

### (5) Confirmation of binding forces of glycan removed antibodies to antigen by using Biacore

First, as an antibody sample, N297S prepared in (1) of Test Example 5 was used. In addition, as a comparison subject, WT prepared in (1) of Test Example 5 was used. For these, the binding amount of each antibody and the antigen was calculated in the same manner as in (2) of Test Example 2.

Table 8 given below shows the binding amount (mAb (RU)) of the antibody when each antibody sample was used for the antigen solution at each concentration, the binding amount (CRP (RU)) of the antigen, the ratio of the binding amount of the antigen when the binding amount of each antibody was considered as 100% (CRP (RU)/mAb (RU)×100, CRP/mAb(%)), as well as the ratio of CRP/mAb in using N297S when CRP/mAb in using WT was considered as 100% (CRP/mAb value ratio (%)).

**[Table 8]**

| Antigen solution concentration | Antibody sample | mAb (RU) | CRP (RU) | CRP/mAb | CRP/mAb value ratio |
|---|---|---|---|---|---|
| 6.3 nM | WT | 460.4 | 36.5 | 7.9% | 103.1% |
| | N297S | 452.7 | 37.0 | 8.2% | |
| 12.5 nM | WT | 460.6 | 60.9 | 13.2% | 103.1% |
| | N297S | 454.7 | 62.0 | 13.6% | |
| 25 nM | WT | 459.8 | 100.2 | 21.8% | 102.8% |
| | N297S | 455.3 | 102.0 | 22.4% | |
| 50 nM | WT | 459.2 | 156.7 | 34.1% | 101.8% |
| | N297S | 457.2 | 158.9 | 34.8% | |
| 100 nM | WT | 460.0 | 221.5 | 48.2% | 101.4% |
| | N297S | 456.6 | 223.0 | 48.8% | |
| Average of CRP/mAb value ratios | | | | | 102.5% |

As shown in Table 8, the CRP/mAb value was higher when N297S from which a glycan was removed was used than when WT from which a glycan was not removed was used, so that it was confirmed that the reactivity (binding force) with an antigen was improved.

### (6) Supporting of antibodies on latex particle carrier

By a publicly-known method, each recombinant antibody (N297S (Example 4) or N297C-S298C (Example 5)) prepared in (1) and polystyrene latex were mixed to support each recombinant antibody on the latex surface (a prepared solution comprising the obtained latex particles supporting the N297S (or N297C-S298C) is referred to as a "N297S (or N297C-S298C)-supporting latex emulsion"). In addition, as a comparison subject, WT (Comparative Example 4) prepared in (1) was treated in the same manner to prepare latex particles supporting WT (a prepared solution comprising the obtained latex particles supporting WT is referred to as a "WT-supporting latex emulsion"). Moreover, as a reference, the glycan removed antibody (Hybridoma glycan removed antibody) and the untreated antibody (Hybridoma-untreated antibody) prepared in (1) of Test Example 1 were treated in the same manner to obtain a glycan removed antibody-supporting latex emulsion and an untreated antibody-supporting latex emulsion, respectively.

### (7) Reactivity of measurement system utilizing latex particles supporting glycan removed antibody or untreated antibody

For the recombinant antibody-supporting latex emulsion, the WT-supporting latex emulsion, the glycan removed antibody-supporting latex emulsion, and the untreated antibody-supporting latex emulsion prepared in the above-described (6), the reactivity with CRP was compared. First as a first reagent, a reagent comprising a Good's buffer and a reaction modifier was prepared. As a second reagent, a reagent comprising a Good's buffer, a reaction modifier, and each antibody-supporting latex emulsion was prepared. In addition, antigen samples were prepared by diluting CRP antigen to concentrations of 0.5, 2, 4, 20, 40, 50, and 100 mg/dL.

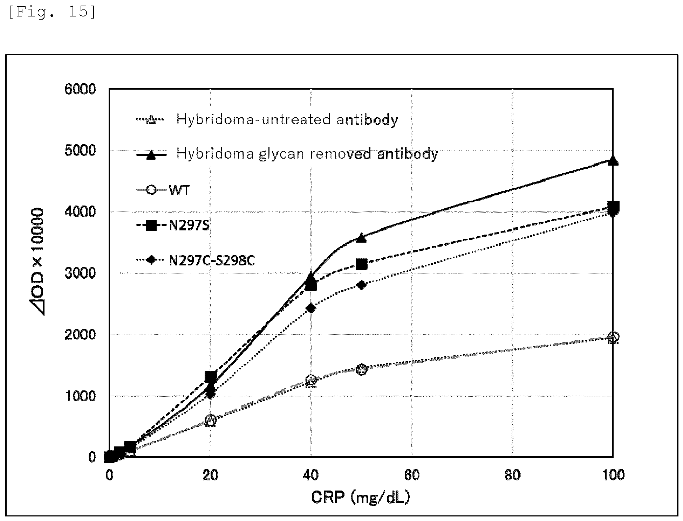

Subsequently, to 80 µL of the first reagent, an equal amount of the and second reagent and the 1.6 µL of the antigen samples prepared at various concentrations were added, and the reaction was measured by an agglutination method, that is, as OD values by using automatic analyzer 7180 (manufactured by Hitachi High-Tech) (main wavelength: 570 nm, sub wavelength: 800 nm). From the OD value measured using each antibody-supporting latex emulsion at each antigen sample concentration, a ΔOD value was calculated in accordance with the following formula: the ΔOD value=the OD value at a wavelength of 570 nm-the OD value at a wavelength of 800 nm. The relations between the obtained ΔOD values (ΔOD×10000) and the antigen sample concentrations are shown in Table 9 given below and Fig. 15.

**[Table 9]**

| Antigen sample (mg/dL) | Antibody-supporting latex emulsion (ΔOD×10000) | | | | |
|---|---|---|---|---|---|
| | Hybridoma-untreated antibody | Hybridoma glycan removed antibody | WT | N297S | N297C-S298C |
| 0 | 6 | 6 | 8 | 8 | 7 |
| 0.5 | 9 | 20 | 11 | 20 | 16 |
| 2 | 53 | 83 | 48 | 80 | 70 |
| 4 | 103 | 162 | 97 | 169 | 145 |
| 20 | 589 | 1162 | 606 | 1312 | 1032 |
| 40 | 1220 | 2943 | 1267 | 2802 | 2430 |
| 50 | 1458 | 3588 | 1433 | 3150 | 2809 |
| 100 | 1940 | 4849 | 1966 | 4084 | 3993 |

As shown in Table 9 and Fig. 15, the ΔOD values were higher when N297S and N297C-S298C from which glycans were removed were used than when WT from which a glycan was not removed was used, so that it was confirmed that the reactivity (binding force) with an antigen was improved as in the comparison between the glycan removed antibody (Hybridoma glycan removed antibody) that was subjected to deglycosylation by enzyme treatment and the untreated antibody (Hybridoma-untreated antibody) that was not subjected to the deglycosylation.

### (Test Example 6) Evaluation of reactivity of mouse anti-human CRP monoclonal antibody (enzyme-treated antibody) from which a glycan was removed by using enzyme (galactosidase treatment or EndoF2 treatment)

### (1) preparation of antibody from which a glycan was removed (galactosidase treatment)

To 1 mg of a mouse anti-human CRP (C-reactive protein) monoclonal antibody (IgG1), 1 µL of β1-4 Galactosidase (produced by QA-bio) was added, followed by keeping at 37°C for 18 hours to prepare a mouse anti-human CRP monoclonal antibody subjected to the deglycosylation treatment (in Test Example 6, referred to as a "galactosidase-treated antibody").

### (2) Preparation of antibody from which a glycan was removed (EndoF2 treatment)

To 1 mg of a mouse anti-human CRP (C-reactive protein) monoclonal antibody (IgG1), 10 µL of EndoF2 (produced by New England Biolabs) was added, followed by keeping at 37°C for 18 hours to prepare a mouse anti-human CRP monoclonal antibody subjected to the deglycosylation treatment (in Test Example 6, referred to as an "EndoF2-treated antibody").

### (3) Glycan analysis of antibodies

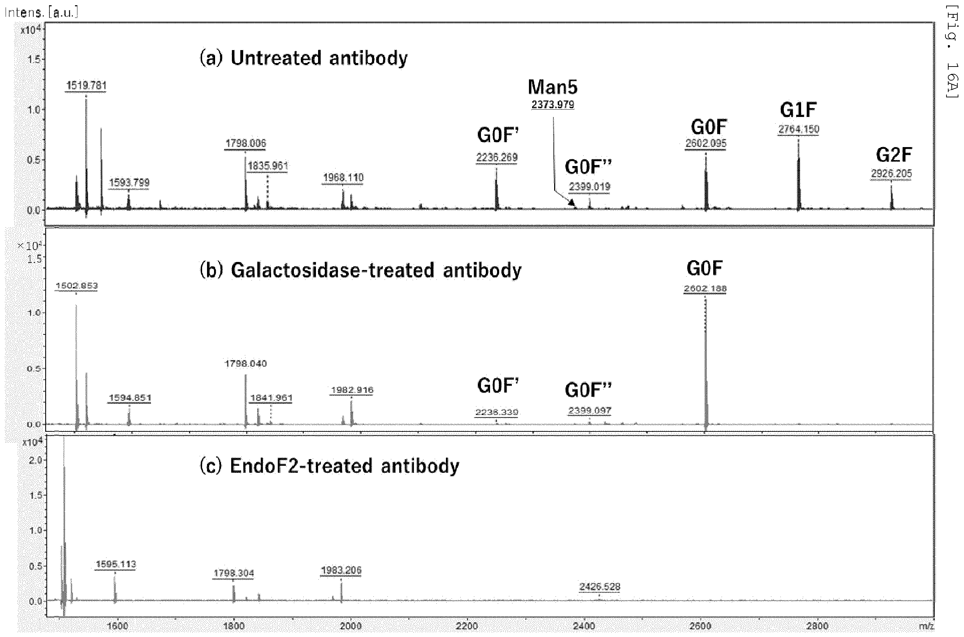

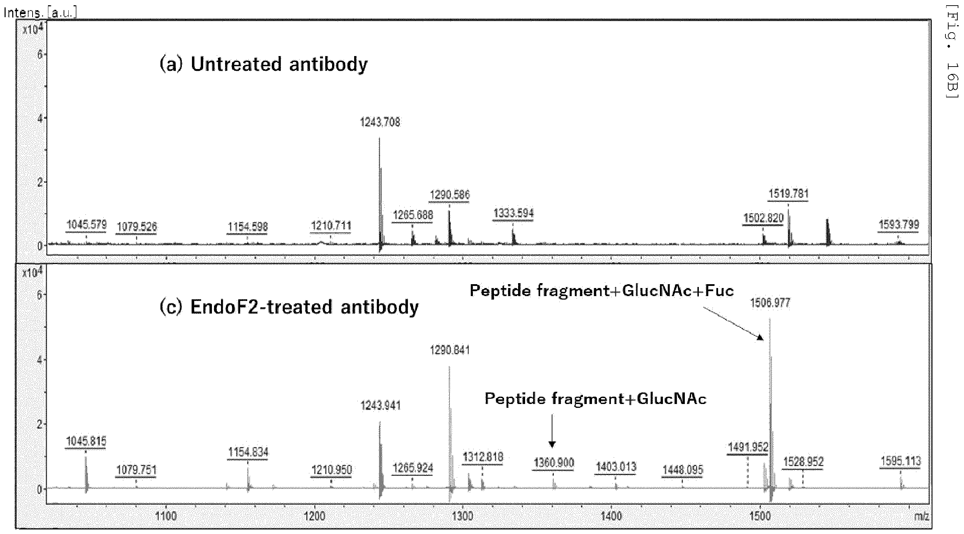

For each enzyme-treated antibody obtained in the above-described (1), (2), glycan analysis was conducted by MALDI-TOF/MS in accordance with the same method as in (2) of Test Example 1. The result of TOF/MS analysis of the galactosidase-treated antibody is shown in (b) of Fig. 16A, and the result of TOF/MS analysis of the EndoF2-treated antibody is shown in (c) of Figs. 16A and 16B. In addition, as a subject, for a mouse anti-human CRP monoclonal antibody (untreated antibody) that was not subjected to the deglycosylation treatments of (1) and (2), the result of TOF/MS analysis obtained by conducting the glycan analysis in the same manner is shown in (a) of Fig. 16A and Fig. 16B. Moreover, each peak position (m/z) and a signal-to-noise ratio (S/N) thereof are shown in Table 10 given below. The schematic diagrams of the basic structures of the N-linked glycan and the partially removed glycan corresponding to each peak are as shown in the above-described Fig. 1A and Fig. 1B.

**[Table 10]**

| | *m*/*z* | | | S/N | | |
|---|---|---|---|---|---|---|
| | Untreated antibody | Galactosidase -treated antibody | EndoF2-treated antibody | Untreated antibody | Galactosidase -treated antibody | EndoF2-treated antibody |
| Peptide fragment | - | - | - | - | - | - |
| Peptide fragment+GlcNAc | - | - | 1360.900 | - | - | 175 |
| Peptide fragment+GlcNAc , Fuc | - | - | 1506.977 | - | - | 3757 |
| Peptide fragment+GOF' | 2236.269 | 2236.339 | - | 121 | 5 | - |
| Peptide fragment+Man5 | 2373.979 | - | - | 8 | - | - |
| Peptide fragment+G0F" | 2399.019 | 2399.097 | - | 29 | 30 | - |
| Peptide fragment+G0F | 2602.095 | 2602.188 | - | 209 | 1260 | - |
| Peptide fragment+G1F | 2764.150 | - | - | 303 | - | - |
| Peptide fragment+G2F | 2926.205 | - | - | 107 | - | - |

As shown in Table 10 and Fig. 16A, in the galactosidase-treated antibody (antibody composition), peaks at 2373.979 m/z (Man5), 2764.150 m/z (G1F), and 2926.205 m/z (G2F) which were observed in the untreated antibody (antibody composition) were not observed, and observed was an increase in peak corresponding to 2602.095 m/z (G0F) which was observed in the untreated antibody (antibody composition). From this, it was confirmed that a glycan partially removed antibody in which at least the N-linked glycans of G1F and G2F in the Fc region of the mouse anti-human CRP monoclonal antibody were partially removed (cleaved and removed by galactosidase) which had G0F as a partially removed glycan was obtained by the deglycosylation treatment of (1) .

In addition, as shown in Table 10, Fig. 16A, and Fig. 16B, in the EndoF2-treated antibody (antibody composition), peaks of G0F', Man5, G0F'', G0F, G1F, and G2F which were observed in a range of 2236.269 to 2926.205 m/z of the untreated antibody (antibody composition) were not observed. In addition, in the EndoF2-treated antibody (antibody composition), peaks at 1360.900 m/z and 1506.977 m/z which were not observed in the untreated antibody (antibody composition) were observed, so that it was confirmed that these peaks were of "GlcNAc" and "GlcNAc+Fuc" binding to the Fc region of the mouse anti-human CRP monoclonal antibody. From this, it was confirmed that the glycan partially removed antibody in which at least the N-linked glycans of Man5, G0F, G1F, and G2F of the Fc region of the mouse anti-human CRP monoclonal antibody were partially removed (cleaved and removed by EndoF2) which had GlcNAc or GlcNAc+Fuc as a partially removed glycan were obtained by the deglycosylation treatment of (2).

In addition, from the result of the glycan analysis of the antibodies, in the obtained galactosidase-treated antibody (antibody composition), an antibody having glycans comprising galactose was not observed. Moreover, the obtained galactosidase-treated antibody (antibody composition) had such a significantly high homogeneity that the relative priority of the antibody having G0F (Pi: the amount of the antibody having G0F/the total antibody amount) was 0.95 and the diversity index (Simpson's diversity index) was 0.05. In addition, the obtained EndoF2-treated antibody (antibody composition) had such a significantly high homogeneity that the relative priority of the antibody having GlcNAc or GlcNAc+Fuc (Pi: the amount (total amount) of the antibody having GlcNAc or GlcNAc+Fuc/the total antibody amount) was 0.91 and the diversity index (Simpson's diversity index) was 0.09. On the other hand, the untreated antibody (antibody composition) had such a significantly low homogeneity that the diversity index (Simpson's diversity index) was 0.73.

### (3) Supporting of antibodies on latex particle carrier

By a publicly-known method, the galactosidase-treated antibody (Example 6) prepared in (1) or the EndoF2-treated antibody (Example 7) prepared in (2) and polystyrene latex were mixed to support the galactosidase-treated antibody or the EndoF2-treated antibody was supported on the latex surface (a prepared solution comprising the obtained latex particles supporting the galactosidase-treated antibody (or the EndoF2-treated antibody) is referred to as a "galactosidase-treated antibody (or EndoF2-treated antibody)-supporting latex emulsion"). In addition, as a comparison subject, the above-described untreated antibody (Comparative Example 1) was treated in the same manner to prepare latex particles supporting the untreated antibody (a prepared solution comprising the obtained latex particles supporting the untreated antibody is referred to as an "untreated antibody-supporting latex emulsion"). Moreover, as a reference, the glycan removed antibody (in Test Example 6, referred to as a "glycopeptidase-treated antibody") prepared in (1) of Test Example 1 was treated in the same manner to obtain a glycopeptidase-treated antibody-supporting latex emulsion.

### (4) Reactivity of measurement system utilizing latex particles supporting glycan removed antibody or untreated antibody

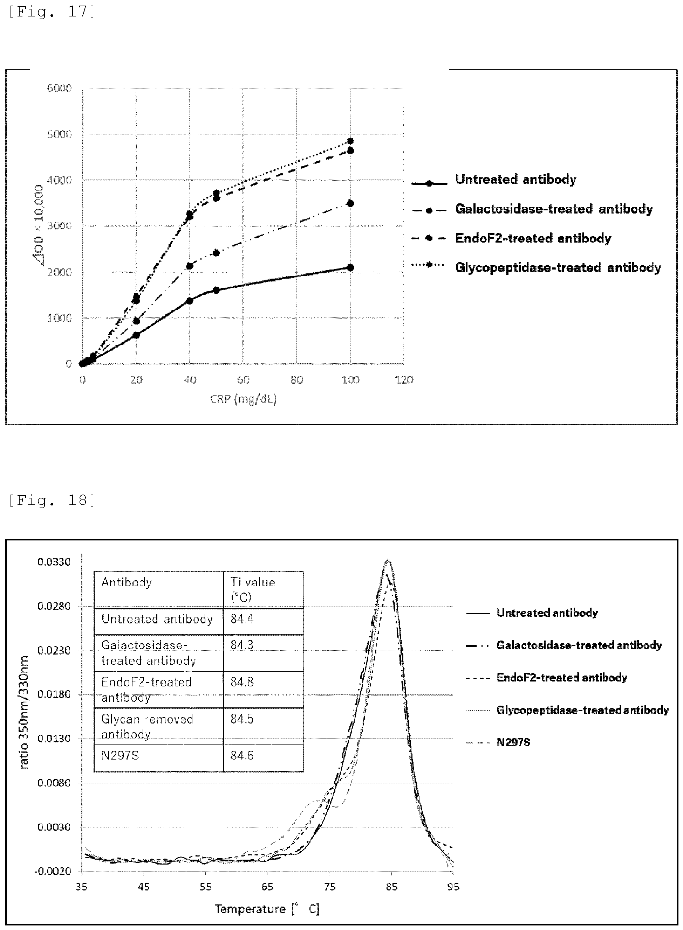

For each of the galactosidase-treated antibody-supporting latex emulsion, the EndoF2-treated antibody-supporting latex emulsion, the untreated antibody-supporting latex emulsion, and the glycopeptidase-treated antibody-supporting latex emulsion prepared in (3), the reactivity with CRP was compared in the same method as in (4) of Test Example 1. The relations between the ΔOD values (ΔOD×10000) and the antigen sample concentrations (CRP (mg/dL) are shown in Table 11 given below and Fig. 17. Table 11 also shows the ratio of the ΔOD value (enzyme-treated antibody ΔOD) (enzyme-treated antibody ΔOD/untreated antibody ΔOD×100, OD value ratio (%)) measured and calculated by using the galactosidase-treated antibody-supporting latex emulsion, the EndoF2-treated antibody-supporting latex emulsion, or the glycopeptidase-treated antibody-supporting latex emulsion when the ΔOD value (untreated antibody ΔOD) measured and calculated by using the untreated antibody-supporting latex emulsion is considered as 100%.

**[Table 11]**

| Antigen sample (mg/dL) | Antibody-supporting latex emulsion (ΔOD×10000) | | | | ΔOD value ratio | | |
|---|---|---|---|---|---|---|---|
| | Untreated antibody | Galactosi dase-treated antibody | EndoF2-treated antibody | Glycopepti dase-treated antibody | Galactosi dase-treated antibody | EndoF2-treated antibody | Glycopepti dase-treated antibody |
| 0 | 5 | 3 | 4 | 4 | - | - | - |
| 0.5 | 12 | 21 | 24 | 23 | 175% | 200% | 192% |
| 2 | 52 | 69 | 84 | 80 | 133% | 162% | 154% |
| 4 | 101 | 131 | 170 | 167 | 130% | 168% | 165% |
| 20 | 632 | 946 | 1478 | 1379 | 150% | 234% | 218% |
| 40 | 1375 | 2141 | 3198 | 3271 | 156% | 233% | 238% |
| 50 | 1608 | 2434 | 3611 | 3722 | 151% | 225% | 231% |
| 100 | 2098 | 3510 | 4651 | 4848 | 167% | 222% | 231% |

As shown in Table 11 and Fig. 17, with 40 mg/dL or more of the antigen sample, for example, the ΔOD value, that is, the reactivity (the binding capability with the antigen) was improved about 1.5 times or more when the galactosidase-treated antibody-supporting latex emulsion was used as compared with when the untreated antibody-supporting latex emulsion was used. In addition, with 40 mg/dL or more of the antigen sample, for example, the reactivity was improved about 2.3 times or more when the EndoF2-treated antibody-supporting latex emulsion was used than when the untreated antibody-supporting latex emulsion was used.

In addition, the reactivity when the EndoF2-treated antibody-supporting latex emulsion was used was higher than the reactivity when the galactosidase-treated antibody-supporting latex emulsion was used, and was slightly lower than or comparable with the reactivity when the glycopeptidase-treated antibody-supporting latex emulsion was used. From these, it was confirmed that the reactivity with an antigen was the highest in an antibody (glycopeptidase-treated antibody) from which all the N-linked glycan at position 297 based on the Kabat numbering in the Fc region was removed, and was higher in an antibody (EndoF2-treated antibody) from which part of the N-linked glycan was removed and shorter glycan remains than an antibody (galactosidase-treated antibody) from which part of the N-linked glycan was removed and longer glycan remains.

### (5) Confirmation of thermal stabilities

The thermal denaturation curves of the galactosidase-treated antibody prepared in (1) and the EndoF2-treated antibody prepared in (2) were obtained and the inflection-point temperatures (Ti) were determined by the same method as in (1) of Test Example 2. In addition, as a comparison subject, for the untreated antibody as well, the thermal denaturation curve was obtained and the inflection-point temperature (Ti) was determined. Moreover, as references, for the glycan removed antibody (glycopeptidase-treated antibody) prepared in (1) of Test Example 1 and the recombinant antibody N297S prepared in (1) of Test Example 5, the thermal denaturation curves were obtained and the inflection-point temperatures (Ti) were determined in the same manner.

The thermal denaturation curves (the vertical axis: the fluorescence intensity ratio between 2 wavelengths (ratio 350 nm/330 nm)) and the Ti values of the galactosidase-treated antibody, the EndoF2-treated antibody, the glycopeptidase-treated antibody, N297S, and the untreated antibody are shown in Fig. 18. It was confirmed that there was hardly a difference in inflection-point temperature (Ti) among all the antibodies. However, in Fig. 18, two peaks of the CH2 region and the Fab region were observed in the EndoF2-treated antibody, the glycopeptidase-treated antibody, and N297S, particularly in N297S, and it was confirmed that the thermal stability of the CH2 region decreased due to the removal of glycans. In contrast, such peaks were not observed in the galactosidase-treated antibody, it was confirmed that the thermal stability of the CH2 region was maintained even when glycans were removed. From these, it was confirmed that the thermal stability was higher in an antibody from which part of the N-linked glycan at position 297 based on the Kabat numbering in the Fc region was removed than an antibody (glycopeptidase-treated antibody, N297S) from which all the N-linked glycan was removed, and the thermal stability was higher in an antibody (galactosidase-treated antibody) from which part of the N-linked glycan was removed and longer glycan remains than in an antibody (EndoF2-treated antibody) from which part of the N-linked glycan was removed and shorter glycan remains. In addition, it was also confirmed that the thermal stability was higher in an antibody (glycopeptidase-treated antibody) in which the amino acid residue at position 297 based on the Kabat numbering in the Fc region was not replaced and is asparagine than in a recombinant antibody (N297S) in which the amino acid residue at the position was replaced.

### [Industrial Applicability]

As described above, the present invention makes it possible to provide an antibody excellent in reactivity with an antigen in immunoassay, an immobilized antibody using the same, an antibody composition, a reagent for immunoassay, and an immunoassay method, as well as a method for improving reactivity of an antibody with an antigen. The present invention makes it possible, for example without immunizing an animal in an immunoassay system to obtain an antibody having stronger binding force to an antigen again or modifying a variable region of the antibody to improve the binding force to the antigen, to enhance the reactivity of antigen-antibody reaction.

[Sequence Listing]IBPF22-529W0-fin.xml

## Claims

1. An antibody for use in an immunoassay method, the antibody comprising an Fc region wherein at least part of a glycan comprised in the Fc region has been removed.

2. The antibody according to claim 1, wherein the Fc region is a region derived from a mammal.

3. The antibody according to claim 1, that is IgG, reduced IgG, or half IgG.

4. The antibody according to claim 1, wherein an amino acid residue at position 297 based on Kabat numbering in the Fc region is asparagine, and part of an N-linked glycan binding to position 297 based on the Kabat numbering has been cleaved and removed.

5. The antibody according to claim 1, wherein an amino acid residue at position 297 based on Kabat numbering in the Fc region is asparagine, and an N-linked glycan has not bound to position 297 based on the Kabat numbering.

6. The antibody according to claim 1, that is a recombinant antibody in which an amino acid residue at position 297 based on Kabat numbering in the Fc region is replaced with an amino acid other than asparagine, and an N-linked glycan has not bound to position 297 based on the Kabat numbering.

7. The antibody according to claim 6, that is a recombinant antibody in which the amino acid residue at position 297 based on the Kabat numbering in the Fc region is replaced with one selected from the group consisting of serine, cysteine, alanine, leucine, isoleucine, valine, glycine, threonine, phenylalanine, tyrosine, methionine, tryptophan, glutamine, and methionine.

8. The antibody according to claim 6, that is a recombinant antibody in which the amino acid residue at position 297 based on the Kabat numbering in the Fc region is replaced with cysteine, and an amino acid residue at position capable of forming a disulfide bond with the cysteine is also replaced with cysteine.

9. The antibody according to claim 8, that is a recombinant antibody in which an amino acid residue at position 298 based on the Kabat numbering in the Fc region is replaced with cysteine.

10. An immobilized antibody comprising: an insoluble carrier; and the antibody according to claim 1 supported on the insoluble carrier.

11. The immobilized antibody according to claim 10, wherein the insoluble carrier is at least one selected from the group consisting of a plate carrier, a membrane carrier, and a particle carrier.

12. An antibody composition comprising the antibody according to claim 1 or the immobilized antibody according to claim 10.

13. A reagent for immunoassay, comprising the antibody according to claim 1 or the immobilized antibody according to claim 10.

14. An immunoassay method comprising a step of using the antibody according to claim 1 or the immobilized antibody according to claim 10.

15. A method for improving reactivity of an antibody with an antigen, comprising a step of removing, in an antibody comprising an Fc region for use in an immunoassay method, at least part of a glycan comprised in the Fc region.
